# EUROPEAN PATENT APPLICATION

(11) **EP 3 964 235 A1**
(43) Date of publication of application: **09.03.2022**
(21) Application number: 20382796.9
(22) Date of filing: 08.09.2020
(51) Int. Cl.: A61K 47/64

(54) **GLYCOCONJUGATES AND MEDICAL USES THEREOF**

(71) Applicant: RemAb Therapeutics SL, 08907 Hospitalet de Llobregat, Barcelona (ES)
(72) Inventor: Olivera Ardid, Sara, 08907 Hospitalet de Llobregat, Barcelona (ES); Bello Gil, Daniel, 08907 Hospitaler de Llobregat, Barcelona (ES)
(74) Representative: Hoffmann Eitle

(57) **Abstract**

The present invention relates to a polymeric glycoconjugate compound comprising a terminal alpha-galactosyl moiety for use in the treatment of anti-aGal IgE antibodies-mediated diseases. It further relates to new polymeric glycoconjugate compounds, a composition comprising thereof, a method for obtaining thereof and its therapeutic or prophylactic use.

## Description

### FIELD OF THE INVENTION

The present invention relates to the field of immunology and immunotherapy. Specifically, it relates to a polymeric glycoconjugate compound comprising a terminal alpha-galactosyl moiety for use in the treatment of anti-αGal IgE antibodies-mediated diseases. It further relates to new polymeric glycoconjugate compounds, a composition comprising thereof, a method for obtaining thereof and its therapeutic or prophylactic use.

### BACKGROUND OF THE INVENTION

Antibodies are essential elements of the humoral immune system (Deal CE, Balazs AB, 2015). However, there are an important number of human pathological conditions in where antibodies trigger or are necessary elements for the disease development. So far, antibody-mediated diseases are mainly related to autoimmune (Wang L *et al.,* 2015), and allergic disorders (Kubo M *et al.,* 2017).

Allergic conditions are disorders mediated by IgE, the antibody isotype that mediates hypersensitivity to a variety of allergens (Hu J *et al.,* 2018). IgE antibodies orchestrate an abnormal adaptive immune response against non-infectious, harmless, exogenous, and environmental substances, including for example glycoproteins from grass, pollen, dust mites, insect venom and food (Hu J *et al.,* 2018).

The number of people affected by such disorders are in a continuous growth globally. Only in Europe, the European Academy of Allergy and Clinical Immunology estimates that more than 50% of its population will suffer from at least one type of allergy by 2025 *(http:*//*www.eaaci.org*/*outreach*/*public-declarations*/*3243-advocacy-manifesto-tackling-the-allergy-crisis-in-europe*)*.* Although the identification of IgE antibodies as a therapeutic target in allergic diseases has been known for many years, the need for curative and preventative treatments remains very high. Plasma exchange, immunosuppressive drugs, and monoclonal antibodies are potential immunotherapies essentially focused to reduce the serological levels of these antibodies (Ring J *et al.,* 2014). Omalizumab (Xolair^{®}), an unspecific treatment directed to the whole population of circulating IgE, is the only anti-IgE therapy approved for treatment of moderate to severe asthma and chronic idiopathic urticaria (Kopp MV, 2011). A concern associated to the unspecific removal of IgE's is that natural IgEs have been described to participate in the physiological host resistance against certain parasites such as arthropods and helminths (Fitzsimmons CM *et al.,* 2014; Mukai K *et al.,* 2016).

In this context, there is increasing evidence of the functional involvement of anti-aGal antibodies in different human disorders (Galili U, 2013). These antibodies, recognized as natural antibodies, target galactose α1-3 galactose (αGal) epitopes and are the most abundant circulating antibody in humans (Galili U, 2015). Primates, including apes, and Old-World monkeys, do not express the αGal epitopes due to an evolutive inactivation of the gene coding for the α1,3-galactosyltransferase enzyme (Galili U, 2015); consequently, they naturally produce these antibodies. There is evidence that links its origin to the gut microbiota (Bello-Gil D *et al.,* 2019). By contrast, the αGal epitope is naturally present on glycolipids and glycoprotein in non-primate mammals (e.g. pigs), prosimians and New-World monkeys. That is the reason why natural anti-αGal antibodies are mainly known for being responsible for the initial rejection of mammalian xenografts exposing this structure (Parker W *et al.,* 1999). Besides, in WO2016026981 A1, the inventors reported that removal of natural anti-aGal antibodies (i.e., of the IgG and IgM isotypes) was useful in the prevention and/or treatment of an infection of bacteria of the gastrointestinal tract. In particular, it was described that removal of anti-αGal antibodies with a polymeric glycoconjugate compound comprising a terminal alpha-galactosyl moiety (i.e., GAS914) resulted in improved bactericidal activity in the serum of the subject. Finally, anti-αGal antibodies have been described to be involved in autoimmune processes, such as in Graves' disease where there is an aberrant expression of the αGal epitope. Similarly, the expression of this epitope by the parasite *Trypanosoma cruzi* induce 'autoimmune like' inflammatory reactions in Chagas' disease (Galili U, 2013). Accordingly, there is a need for finding new agents suitable for treating said diseases. Preferably, agents with improved formulation, pharmacokinetic or pharmacodynamic properties, or easier or less expensive to produce.

It has been reported that anti-aGal IgE antibodies can mediate allergies to mammalian meat inducing a delayed hypersensitivity reaction (Commins SP *et al.,* 2009; Wilson JM and Platts-Mills TAE, 2019) and also an immediate onset anaphylaxis during first exposure to proteins with glycosylation patterns containing the αGal epitope, such as after intravenous administration of the monoclonal antibody cetuximab (Commins SP, Platts-Mills TAE, 2013). Similarly, it has been reported that αGal residues are a potential target of IgE-mediated reactivity to antivenom polyclonal serums from non-primate origin, such as equine antivenom, and a possible cause of the high incidence of hypersensitivity reactions during the first application of equine antivenom (Fischer J *et al.,* 2017). Other authors have reported that IgE antibodies to cat IgA are found in the serum of cat-sensitized human patients, and that these are mainly directed to αGal residues in oligosaccharides modifying cat IgA (Gronlund H *et al.,* 2009). Actually, because αGal is present in muscles, blood cells, parenchymal tissue and secretions of non-primate mammals, allergic reactions can occur upon exposure to a number of mammalian products, such as meat, innards (e.g. kidneys), milk, gelatin, blood products, etc. (Wilson JM and Platts-Mills TAE, 2019). An association between IgE antibodies to αGal and a premature degeneration of bioprosthetic aortic valves has also been suggested (Hilger C *et al.,* 2019).

The prevalence of the αGal syndrome is drastically increasing. Alpha-gal allergy has been reported at least in 18 countries on all six continents, including Australia, United States, Canada, Europe (France, Spain, Germany, Belgium, Switzerland, Sweden, United Kingdom, Italy, and Norway), Asia (Korea and Japan), Central America (Panama), South America (Brazil), and Africa (South Africa and Ivory Coast) (Kwak M *et al.,* 2018, Raghavan RK *et al.,* 2019, Wilson JM and Platts-Mills TAE, 2019). Although it has become an emergent allergy worldwide, the highest incidence is reported in the United States (Commins SP, Platts-Mills TAE, 2013), Australia (Kwak M *et al.,* 2018), and North Europe (Gonzalez-Quintela A. *et al.,* 2014). An initial study suggested that the prevalence of IgE antibodies to αGal only in central Virginia might be approximately 10% (Commins SP *et al.,* 2009). The prevalence of specific IgE to alpha-Gal was 5.5% and 8.1% in a Danish and Spanish study group, respectively (Gonzalez-Quintela A *et al.,* 2014). This population is at risk of developing an allergic process mediated by anti-αGal IgE antibodies.

There is strong evidence that bites from hard ticks of the genus *Ixodes* and *Amblyomma,* like *Amblyomma americanum* (North America), *Ixodes ricinus* (Europe), *Ixodes holocyclus* (Australia), *Amblyomma testudinarium* (Japan) and members of the *Amblyomma cajennense* complex, such as *Amblyomma sculptum* (Panama) are associated with αGal-IgE sensitization (Berg EA *et al.,* 2014; Wilson JM *et al.,* 2017). Moreover, it remains likely that more species of ticks, and even other ectoparasites such as chiggers, are causally related to αGal-sensitization (Wilson JM and Platts-Mills TAE, 2019). Consistent with the premise that tick saliva is involved in sensitization to αGal, Araujo RN *et al.* showed that subcutaneous injection of tick saliva was sufficient to induce αGal-specific IgE in an αGal-knockout mouse model (Araujo RN *et al.,* 2016).

The prevalence of allergic conditions mediated by IgE anti-aGal is expected to increase in the coming years. The habitat of hard ticks is expanding globally, even reaching territories with no presence of these tick species historically (Sagurova I *et al.,* 2019, Sonenshine DE, 2018). Global warming, reforestation, and increases in commerce and travel are critical underlying factors influencing the rate and extent of range expansion for ticks and tick-borne associated diseases (Molaei G *et al.,* 2019).

In addition, anti-αGal IgE antibodies have also been reported to be increased in Lyme disease endemic areas and cannot be discarded that these may play a role in the onset, development or progression of the disease (Tjernberg I *et al.,* 2017). Accordingly, IgE anti-αGal antibodies have been described to be involved in diverse diseases, disorders and conditions, especially in allergic responses to the αGal epitope, generically referred as the αGal syndrome, which prevalence is rapidly increasing, seemingly due to the global expansion of the hard ticks habitat. Therefore, there is a need for finding new treatments for said diseases, disorders, or conditions.

### SUMMARY OF THE INVENTION

Here we describe a novel immunotherapy, based on the intracorporal removal of IgE anti-αGal antibodies further to the administration of polymeric αGal-glycoconjugates to a subject, for the therapeutic treatment or prevention of a disease, disorder or condition where anti-αGal IgE antibodies have been described to be involved in its onset, development, or progression (generically referred herein as an anti-αGal IgE antibodies-mediated disease), more specifically for the therapeutic treatment or prevention of the αGal syndrome. In addition, new polymeric αGal-glycoconjugate compounds are described herein with a lower αGal epitope load with respect to GAS914 which have surprisingly been found to be equally effective in the removal of anti-αGal antibodies, irrespectively of the isotype. Thus, the invention also relates to such polymeric glycoconjugates, its method of manufacturing and the medical uses thereof, including anti-αGal IgE antibodies-mediated diseases, as well as disorders involving other isotypes of anti-αGal antibodies such as in Graves and Chagas' disease or in the therapeutic treatment or prevention of infections caused by bacteria of the intestinal tract.

The use of polymeric glycoconjugates lies in the fact that anti-αGal antibodies have naturally high avidity for multivalent antigens, but low affinity for single oligosaccharides (Katopodis AG *et al.,* 2002).

*In vitro* studies were performed with serum of αGal-sensitized human subjects (Example 2). In agreement with previous research works (Commins SP, Platts-Mills TAE, 2013; Rispens T *et al.,* 2013), human subjects showed augmented levels of anti-αGal IgE compared to controls (healthy volunteers). Interesting, we also found the same trend for the rest of isotypes assessed (IgM, IgG, IgA).

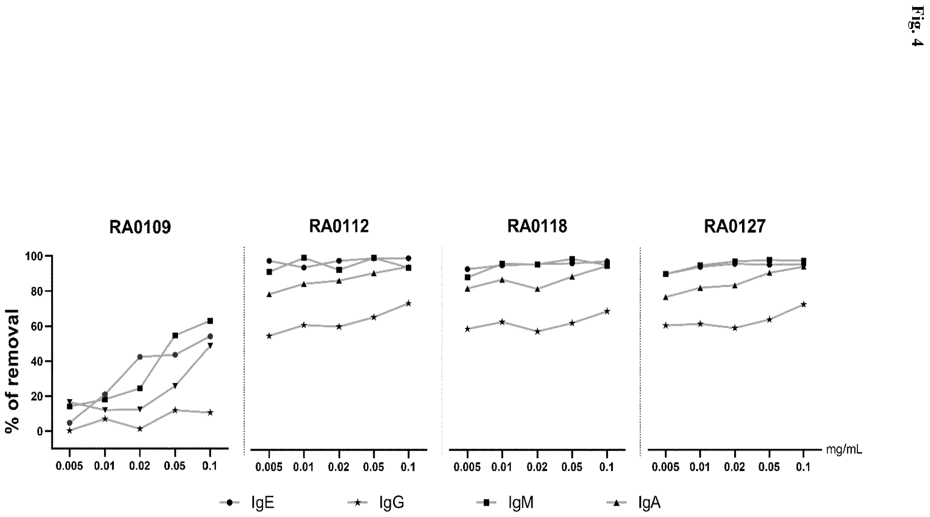

We further demonstrated the efficacy of the polymeric αGal-glycoconjugates described herein for inhibiting *in vitro* and *in vivo* the anti-αGal IgE antibodies. In Example 2, the inventors observed a gradual-linear increase in anti-αGal antibodies inhibition as a function of glycoconjugate concentration. Despite the different αGal-load, RA0112 (12%), RA0118 (18%), and RA0127 (27%) glycoconjugates showed an unexpected similar trend. For IgE and IgM isotypes, a very high percentage of removal (greater than 90%) was detected at the lowest concentration assessed, reaching almost 100% of inhibition from 0.02 mg/mL. For anti-αGal IgA, the inhibition was around 80% at 0.005 mg/mL, reaching more than 95% of IgA removal at 0.1 mg/mL. Finally, the removal of anti-αGal IgG reached approximately 75% at the highest concentration of glycopolymer tested (Fig. 4). It was surprising to find RA0112 (less than 50% of saccharide derivatization compared to RA0127) and RA0118 glycoconjugate (1/3 reduction of glycan load respect to RA0127) as the best inhibitors for some of the anti-αGal isotypes and concentrations evaluated, although without significant differences with respect to RA0127. This result was completely unexpected.

Additionally, for all the glycoconjugates and the assessed concentrations, the exposure to the polymeric glycoconjugates mostly inhibited anti-αGal IgE and IgM isotypes (very similar trends) with a lower inhibition effect on the IgA and IgG isotypes, respectively. Due to the affinity maturation and class switching processes, IgG and IgA antibodies typically have substantially higher affinities than IgM antibodies for protein antigens (single binding sites). However, this behavior changes for molecules with many glycan antigens (exposing repetitive binding sites), where IgM outcompetes IgG and IgA in serum (Muthana SM *et al.,* 2015). IgM molecules form pentamers whose ten antigen-binding sites can attach simultaneously to multivalent antigens like carbohydrates. The relatively low affinity of the IgM is compensated by the multipoint binding that confers a high overall avidity to the IgM isotype for glycan antigens. In contrast, IgG and IgA isotypes are monomeric with only two binding sites (Eisen HN, 2014). Therefore, when using glycopolymers exposing multiple antigenic binding sites, it is generally expected to achieve higher inhibition rates for IgM isotype compared to IgG and IgA isotypes. Because IgE is a monomeric antibody form, like IgG and IgA in serum, a similar inhibition trend may *a priori* be expected for IgE and IgG, and not for IgM and IgE. Accordingly, it was completely unexpected that anti-αGal IgE were found by the inventors to be inhibited more efficiently by the glycoconjugates of the invention than IgG and IgA and to show a similar behavior to IgM isotype.

The *in vitro* proof of concept was validated *in vivo* (Example 3). The inventors developed a model to αGal sensitization in GalT-KO mice, achieving a clear induction of the IgE anti-αGal antibodies, together with IgG and IgM. As shown in the animal model described in Example 3, the increase in the levels of IgE and IgG anti- αGal antibodies was shown to be correlated with the total serological levels of IgE and IgG2b isotype. RA0118 showed a high efficacy as therapy for intracorporeally removing IgE anti-αGal antibodies.

Accordingly, the first aspect of the invention relates to a polymeric glycoconjugate compound comprising a terminal alpha-galactosyl moiety, wherein said compound comprises a poly-L-lysine backbone and is represented by formula (I): 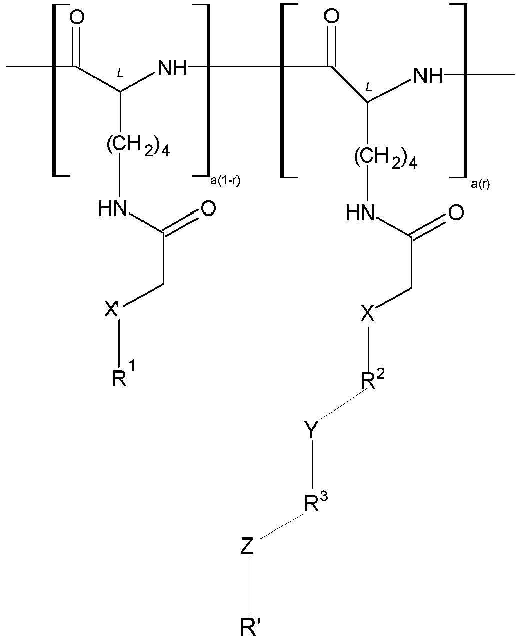
wherein R¹ is - hydrogen, alkyl, alkenyl, alkynyl, alkoxy, acyl, amine, carboxyl, carboxyl ester, cycloalkyl, cycloalkenyl, heterocycloalkyl, heterocycloalkenyl, aryl, and heteroaryl;
X, X', and Y are each independently -C(O)-, -C(O)O-, -C(O)NR-, -O-, -S-, or -NR-;
Z is a direct bond, -O-, or -C(O)NR-;
R² is -(CH₂)ₘ, -RNC(O)(CH₂)ₙC(O)NR-, -(CH₂)ₚC(O)NH(CH₂)_{q}- or -C(O)(CH₂)ₛC(O)-;
R³ is -(CH₂)ₜ-;
each occurrence of R is independently selected from the group consisting of hydrogen, OH, alkyl, alkenyl, alkynyl, alkoxy, acyl, carboxyl, carboxyl ester, cycloalkyl, cycloalkenyl, heterocycloalkyl, heterocycloalkenyl, aryl, and heteroaryl;
R' is an oligosaccharide, preferably an oligosaccharide with 2 to 6 monosaccharide units, with terminal alpha-galactosyl moiety at the non-reducing end; and
wherein m, n, p, q, s and t are each independently an integer of 1-6. Illustrative non-limitative examples are: methyl, ethyl, propyl, isopropyl, butyl, isobutyl, sec-butyl, tert-butyl, n-pentyl, neo-pentyl and n-hexyl.

In a second aspect, the invention refers to a method for obtaining a compound of formula (I) as described herein.

In a third aspect, the invention relates to a composition comprising the polymeric glycoconjugate compound of formula (I) as described herein.

In a fourth aspect, the present invention relates to a process of preparation of a pharmaceutical composition, said process comprising admixing the compound of formula (I) as described herein with a pharmaceutically acceptable carrier, vehicle, or excipient.

In a fifth aspect, the invention relates to a compound of formula (I) as described herein for use as a medicament.

In a sixth aspect, the invention relates to a compound of formula (I) as described herein, or a pharmaceutical composition as described herein, for use in the treatment of diseases wherein anti-aGal antibodies have been described to be involved in its onset, development, or progression, generically referred herein as a disease mediated by anti-aGal antibodies.

In a related aspect, the present application concerns a method of treating diseases wherein anti-aGal antibodies have been described to be involved in its onset, development, or progression, in a subject wherein said method comprises administering a therapeutically or prophylactically effective amount of a polymeric glycoconjugate compound of formula (I) as described herein to a subject.

In a further related aspect, the present application pertains to the use of a polymeric glycoconjugate compound of formula (I) as described herein for the manufacturing of a medicament for the treatment of diseases wherein anti-aGal antibodies have been described to be involved in its onset, development, or progression.

In an additional aspect, the application relates to a polymeric glycoconjugate compound comprising a terminal alpha-galactosyl moiety for use in a method of treating anti-aGal IgE antibodies-mediated diseases in a subject. In a particular embodiment, said compound comprises a poly-L-lysine backbone and is represented by formula (I): 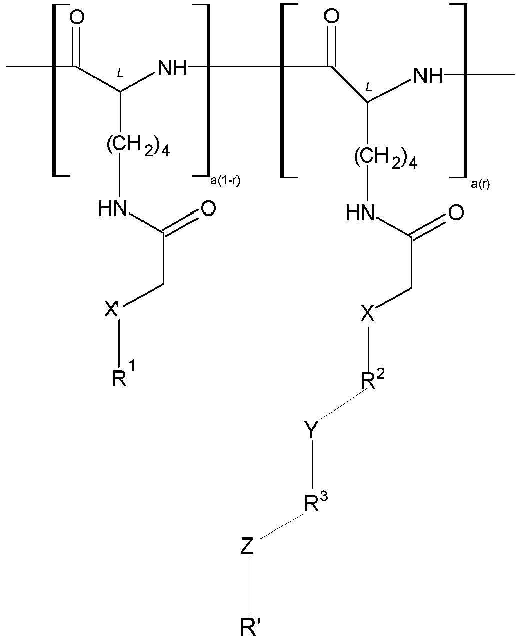
wherein R¹ is - hydrogen, alkyl, alkenyl, alkynyl, alkoxy, acyl, amine, carboxyl, carboxyl ester, cycloalkyl, cycloalkenyl, heterocycloalkyl, heterocycloalkenyl, aryl, and heteroaryl;
X, X', and Y are each independently -C(O)-, -C(O)O-, -C(O)NR-, -O-, -S-, or -NR-;
Z is a direct bond, -O-, or -C(O)NR-;
R² is -(CH₂)ₘ, -RNC(O)(CH₂)ₙC(O)NR-, -(CH₂)ₚC(O)NH(CH₂)_{q}- or -C(O)(CH₂)ₛC(O)-;
R³ is -(CH₂)ₜ-;
each occurrence of R is independently selected from the group consisting of hydrogen, OH, alkyl, alkenyl, alkynyl, alkoxy, acyl, carboxyl, carboxyl ester, cycloalkyl, cycloalkenyl, heterocycloalkyl, heterocycloalkenyl, aryl, and heteroaryl;
R' is an oligosaccharide, preferably an oligosaccharide with 2 to 6 monosaccharide units, with terminal alpha-galactosyl moiety at the non-reducing end; and
wherein m, n, p, q, s and t are each independently an integer of 1-6.

In a related aspect, the present application relates to a method of treating anti-aGal IgE antibodies-mediated diseases in a subject wherein said method comprises administering a therapeutically or prophylactically effective amount of a polymeric glycoconjugate compound, preferably of formula (I) as described herein, to a subject.

In a further related aspect, the present application pertains to the use of a polymeric glycoconjugate compound, preferably of formula (I) as described herein, for the manufacturing of a medicament for the treatment of anti-αGal IgE antibodies-mediated diseases.

### BRIEF DESCRIPTION OF THE DRAWINGS

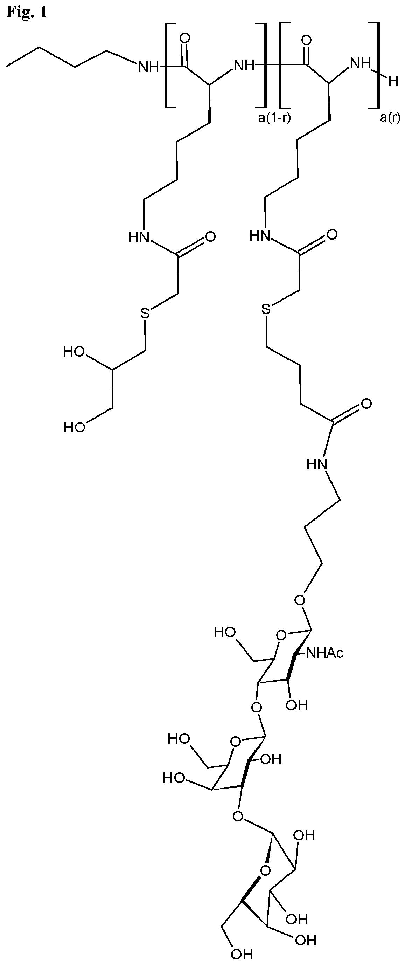

**Figure 1****.** The general structure of the tested polymeric αGal-glycoconjugates. a: 1000 ± 10% l-lysine units; r: 0,09, 0,12, 0,18 or 0,27 (substituted fraction of αGal-epitope).

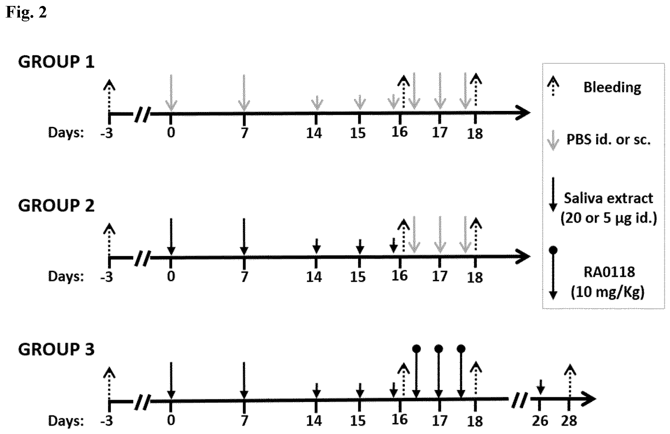

**Figure 2****.** Scheme of GalT-KO mice sensitization for IgE anti-αGal production (not at scale). Group 1: double negative control (PBS). Group 2 and 3 (αGal-sensitized mice) were treated with PBS (control) and RA0118, respectively.

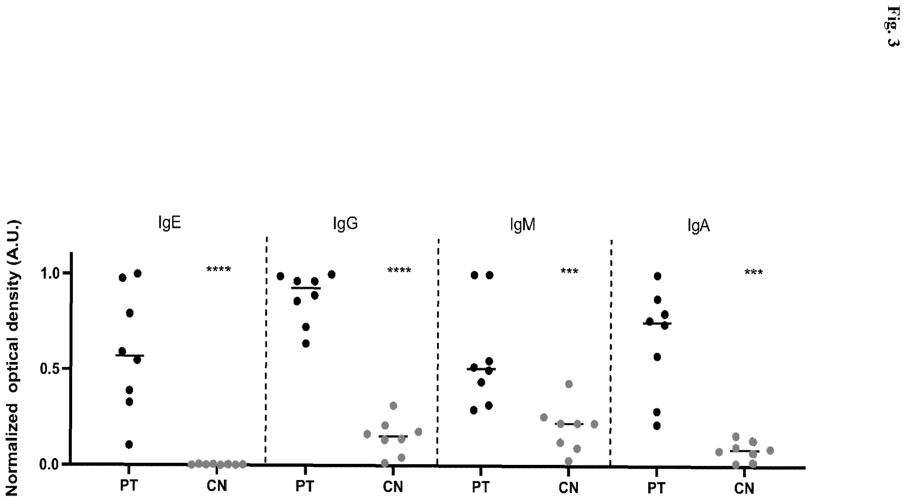

**Figure 3****.** Serological levels of anti-αGal antibodies (IgM, IgG, IgA, and IgE) in αGal-sensitized (PT) and healthy human subjects (CN). Serum was conveniently diluted for ELISA determination as follow, 1:100 for IgM and IgG, 1:25 for IgA, and 1:10 for IgE (n=8). Serum samples were determined by triplicate and individual mean values (OD₄₉₂ₙₘ) were normalized with respect to the maximum signal obtained in each experimental run. The horizontal bars in the graph represent the median in each experimental group and isotype. Differences were considered statistically significant when p < 0.05 (*: p < 0.05; **: p < 0.01; ***: p < 0.001; ****: p < 0.0001).
**Figure 4****.** *In vitro* inhibition of the different isotypes of anti-aGal antibodies in αGal-sensitized human subjects by αGal-glycoconjugates. RA0109, RA0112, RA0118 and RA0127 were individually incubated (mild agitation) at growing concentrations (0.005-0.01-0.02-0.05-0.1 mg/mL) with serum of ten αGal-sensitized during 12 h, at 4°C. Phosphate buffered saline (PBS) was used as a vehicle and negative control. Serum samples were determined by triplicate and individual values (OD₄₉₂ₙₘ) were expressed as median of removal (%), referred to the same serum treated with the vehicle (PBS) under identical conditions (n=10).

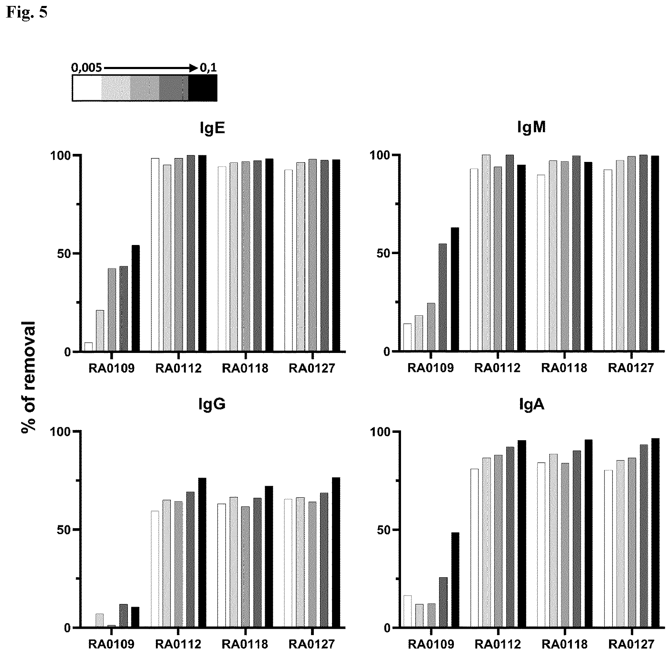

**Figure 5****.** Inhibition of the different anti-αGal antibodies isotypes in αGal-sensitized human subjects at growing concentrations of RA0109, RA0112, RA0118 and RA0127. The bars (from white to black) represent the median expressed as percentage of removal (n=10) at growing glycopolymer concentrations (0.005-0.01-0.02-0.05-0.1 mg/mL).

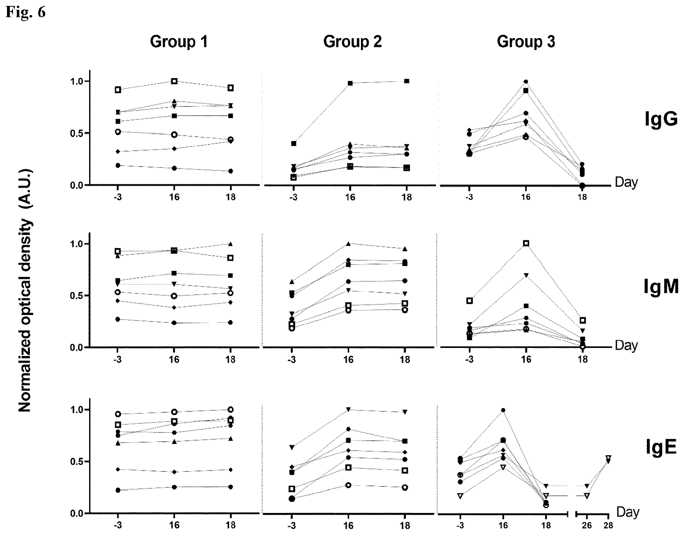

**Figure 6****.** Induction of anti-αGal antibodies in GalT-KO mice (n=7). Group 1 was a double-negative control (PBS). Mice from Group 2 and 3 were sensitized to αGal residues by intradermally immunization of saliva from *Amblyomma sculptum.* The Group 2 was a negative control for the treatment (PBS sc.). In the Group 3, animals were treated with three consecutives subcutaneous RA0118 doses (10 mg/Kg). The induction procedure was repeated in two animals of Group 3 two weeks after last treatment with RA0118 (day 26). Animal blood was collected by controlled-submandibular bleeding on days -3 (baseline), 16 (4 h after induction), 18 (4h after treatment) and 28 (after reinduction, Group 3) for ELISA analysis. Individual serum samples were determined by triplicate and individual values were normalized with respect to the maximum signal obtained in each experimental run.

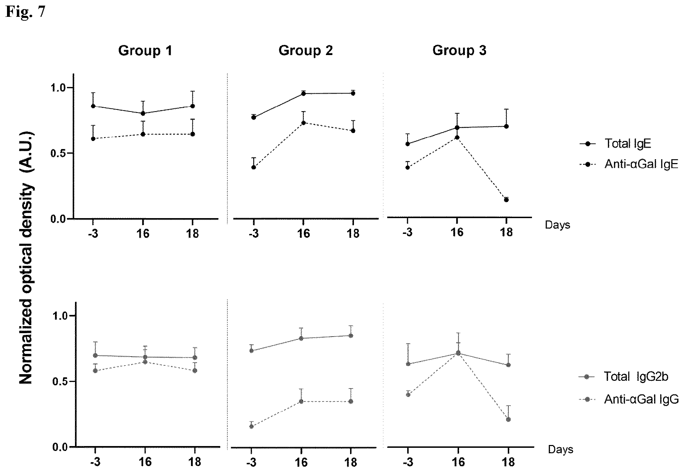

**Figure 7****.** Relation between serological levels of total IgE (continuous line) and anti-aGal IgE (dotted line) antibodies, and total IgG2b (continuous line) and anti-αGal IgG (dotted line) after GalT-KO mice sensitization with *Amblyomma sculptum* saliva. Individual values (optical density units) were normalized with respect to the maximum signal obtained in each experimental run and graphed as mean plus SEM (n=5).

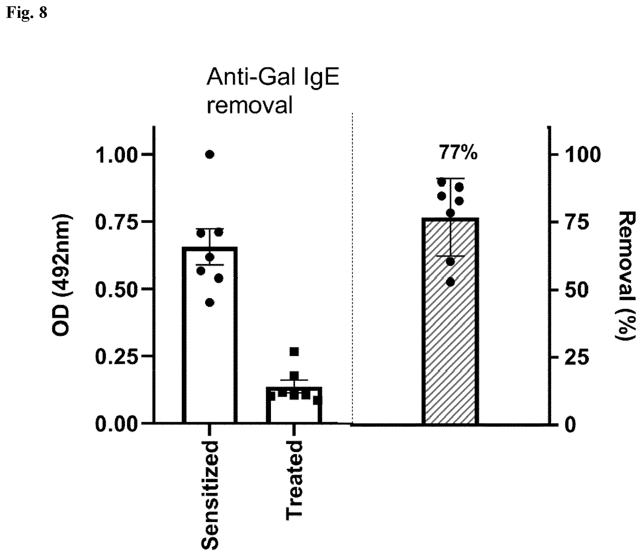

**Figure 8****.** *In vivo* removal of IgE anti-aGal in sensitized GalT-KO mice with RA0118 (10 mg/Kg) (n=7). Mice from Group 3 were sensitized to αGal residues by intradermally immunization of saliva extract from *Amblyomma sculptum.* Animals were then treated with three consecutives subcutaneous RA0118 doses (10 mg/Kg) to inhibit the induced anti-αGal IgE antibodies (according to scheme for Group 3 in Figure 2). Animal blood was collected by controlled-submandibular bleeding on days 16 (sensitized animals) and 18 (treated animals) to assess anti-αGal IgE removal by ELISA. Left: individual serum samples were determined by triplicate and plotted as optical density mean (OD_{492 nm}). Right: anti-αGal IgE removal was expressed as percentage referred to sensitized animals.

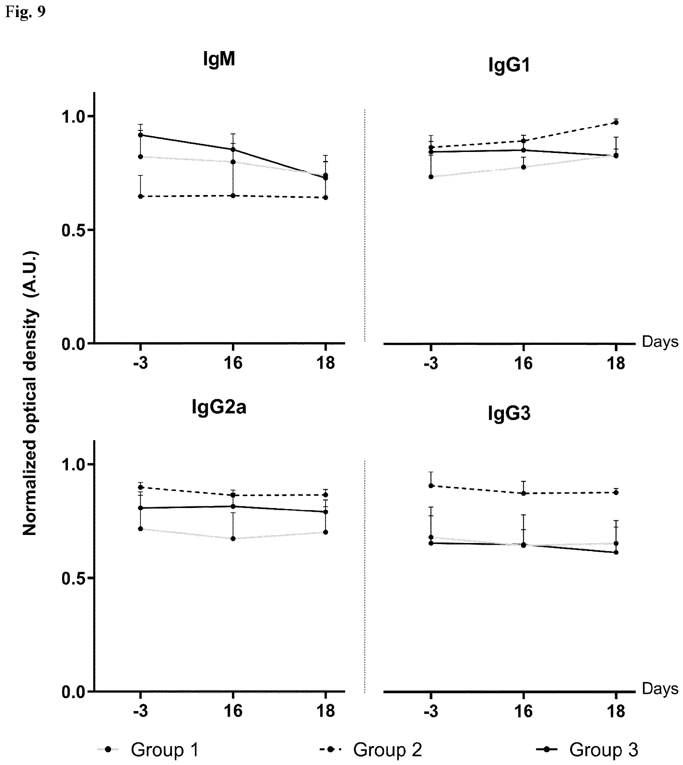

**Figure 9****.** Serological levels of total IgM, IgG1, IgG2a, IgG3 immunoglobulins after GalT-KO mice sensitization with *Amblyomma sculptum* saliva. Individual values (optical density units) were normalized with respect to the maximum signal obtained in each experimental run and graphed as mean plus SEM (n=5).

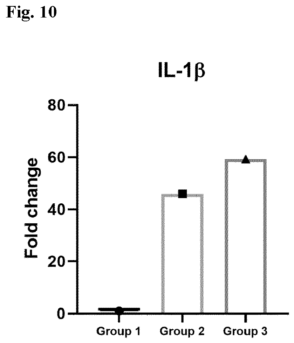

**Figure 10****.** Fold-change of the serological levels of IL-1β on day 16 respect to the baseline (day -3) for the different experimental groups. Serological levels of cytokines were followed by protein array (n=6).

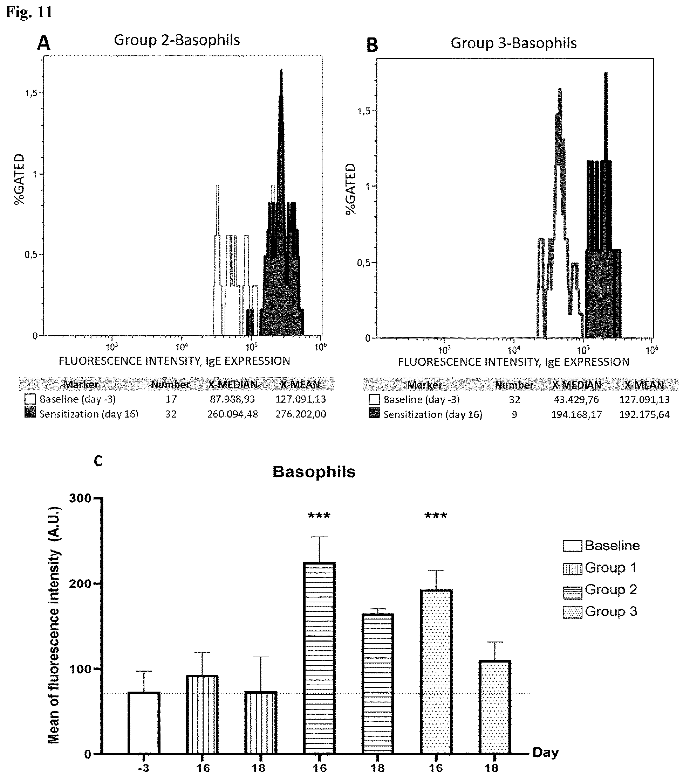

**Figure 11****.** Induction of IgE expression in basophil membranes of GalT-KO mice. It is shown typical histograms obtained for Group 2 (A) and Group 3 (B), and the mean of fluorescence intensity plus SEM for the different experimental groups (C). In every FACS determination of WBC were recorded about 150.000 total events, of which 50.000 were CD45 positive. (n=7).

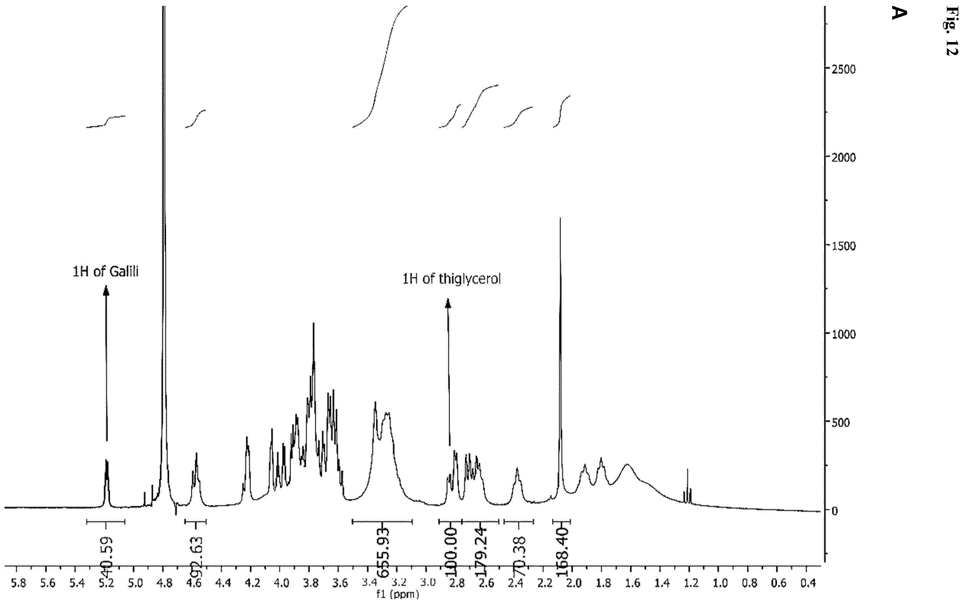

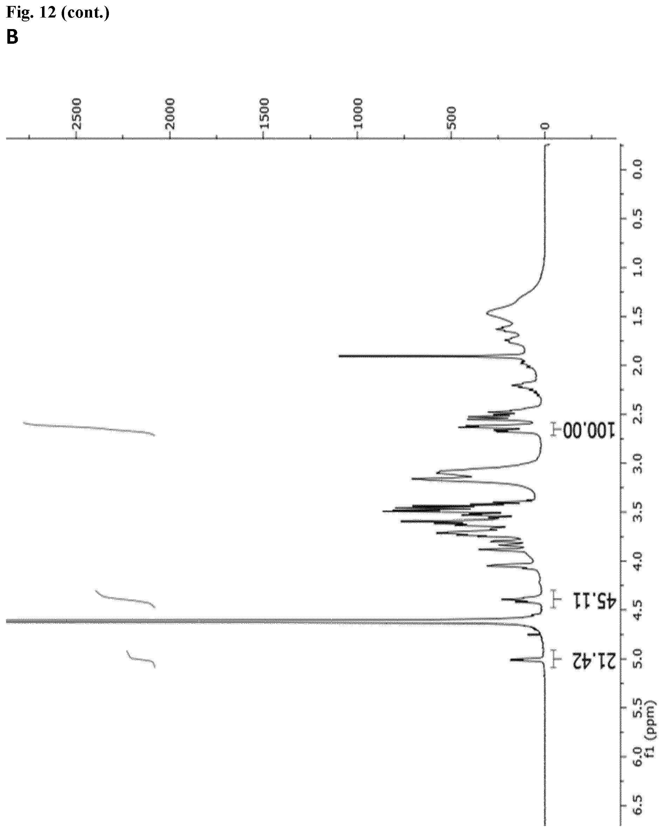

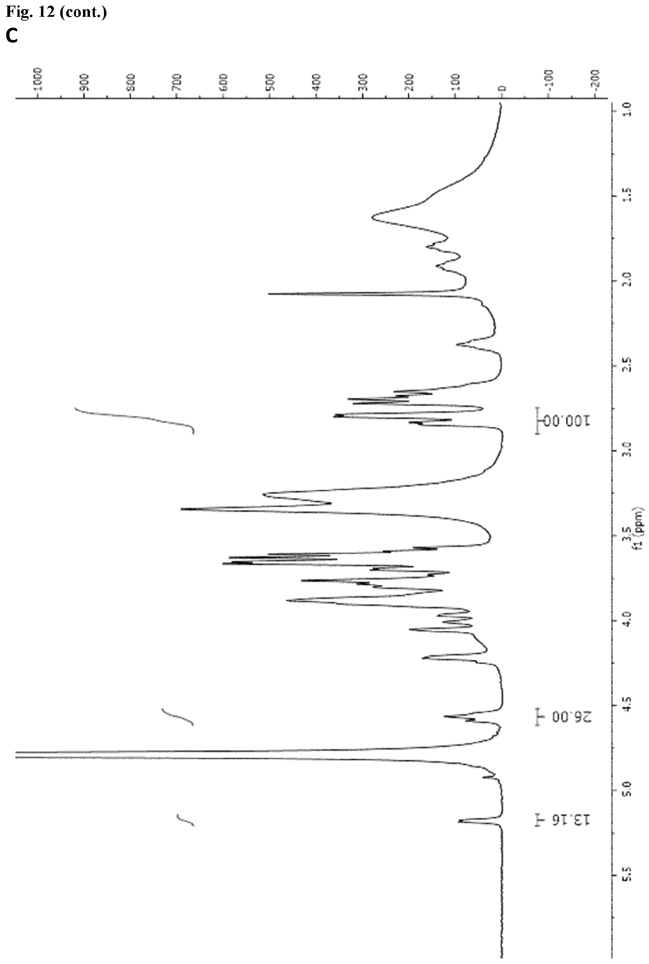

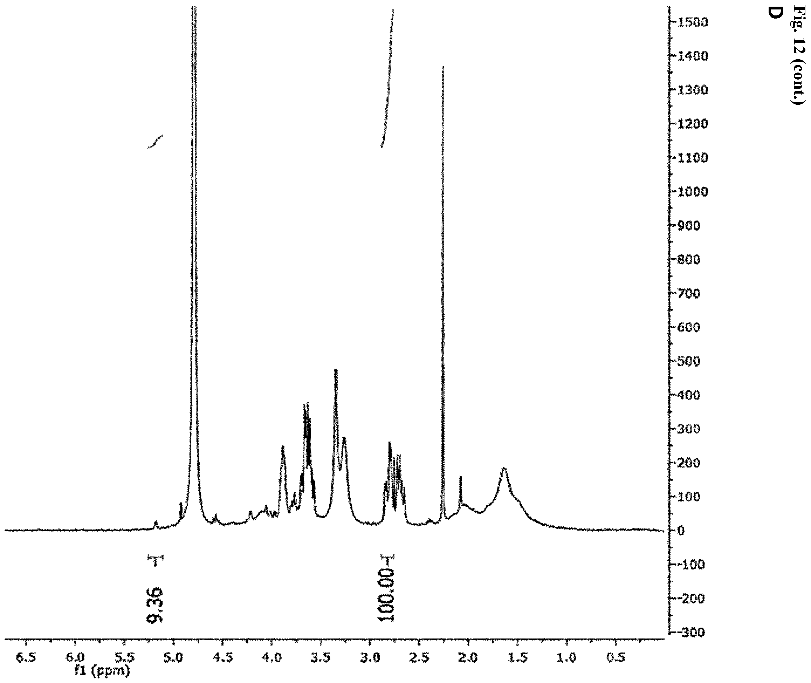

**Figure 12****.** Proton NMR (H) spectrum of the purified polymeric αGal-glycoconjugates. A direct relation between protons characteristic of Galili-epitope and thioglycerol was established. Proton (5.2 ppm) corresponds to Galili-epitope and proton (2.8 ppm) to thioglycerol. The integration of these signals allowed defining the load relation between Galili-epitope and thioglycerol in the polymers. Resulting αGal-glycoconjugates presented twenty-seven (RA0127, Fig. 12A), eighteen (RA0118, Fig. 12B), twelve (RA0112, Fig. 12 C) and nine percentage (RA0109, Fig. 12D) of Galili-epitope load in the final structure.

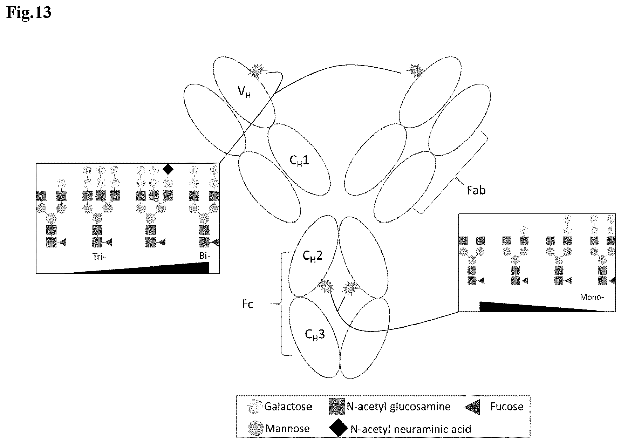

**Figure 13****.** Cetuximab structure and its glycosylation profile. Adapted from Wilson J.M. and Platts-Mills TAE, 2018.

### DETAILED DESCRIPTION OF THE INVENTION

### Definitions

The term "α-galactosyl" or "α-galactosyl moiety" or "α-galactosyl residue", as used herein, relates to a terminal galactose residue in a molecule, i.e., to a glycosyl radical derived from α-galactose. In some contexts, the term alfa-galactosyl is related in particular to a unit of alfa-galactose bound to a second monosaccharide. For instance, said α-galactosyl moiety can be Galα1-3Gal, Galα1-2Gal, Galα1-6Gal or Galα1-6Glc. Preferably, said α-galactosyl moiety is Galα1-3Gal.

The term "agent comprising a terminal α-galactosyl moiety", as used herein, also known as "alpha-galactosyl agent", or "anti-αGal binding agent" refers to any molecule, or part of a molecule, with a terminal structure comprising Galα1-3Galβ1-4GlcNAc-R, Galα1-3Galβ1-3GlcNAc-R, or any carbohydrate chain with terminal Galα1-3Gal at the non-reducing end, or any molecule with terminal α-galactosyl unit, capable of binding the anti-αGal antibody, wherein said α-galactosyl unit may be bound to a second monosaccharide, such as galactose or glucose. The galactose-α-1,3-galactose (Galα1-3Gal) residue (also known as "α-Gal epitope", "alpha-galactosyl epitope", or "anti-aGal binding epitope") is synthesized by the glycosylation enzyme α-1,3-galactosyltransferase (α1,3GT) and it is expressed in very large amounts on the cells of non-primate mammals, prosimians and in New-World monkeys. The α1,3GT gene was inactivated in ancestral Old-World primates. Thus humans, apes, and Old-World monkeys lack α-gal epitopes and produce high titer anti-αGal antibodies.

The term "subject" or "patient" as used herein, refers to mammals. Mammalian species that can benefit from the disclosed methods of treatment include any species which generates anti-αGal antibodies. This may include humans, and non-human Old-World primates, such as apes, and Old-World monkeys, including *inter alia* chimpanzees and orangutans. In a preferred embodiment, refers to humans, including children and adults.

As used herein, "treatment", "treating" or "treat" refer to: (i) preventing a disease, disorder or condition from occurring in a subject which may be predisposed to the disease, disorder and/or condition but has not yet been diagnosed as having it. In certain embodiments, such term refers to the prevention of the disease in a subject which IgE levels or anti-αGal IgE levels have been found to be elevated but prior to a second or further exposure to the αGal epitope; (ii) inhibiting the disease, disorder or condition, i.e., arresting or slowing down its development or progression; and/or (iii) relieving the disease, disorder or condition, i.e., causing regression of the disease, disorder and/or condition. In certain embodiments, such term refers to the amelioration or eradication of a disease or symptoms associated with a disease. As used herein, the term "preventing" does not include "inhibiting" or "relieving" the disease, disorder, or condition.

As used herein a "therapeutically or prophylactically effective amount" refers to an amount effective, at dosages and for periods of time necessary to achieve the desired therapeutic or prophylactic result, such as one or more of the following results: a significant delay of the onset or progression of the disease; a significant reduction of the severity of one or more symptoms, such as urticaria, angioedema or anaphylaxis; a significant reduction of anti-αGal IgE antibodies in serum, or a significant attenuation of mortality associated to an immune reaction and/or allergic response.

As used herein an "anti-αGal IgE antibodies-mediated disease" refers to a disease, disorder, or condition where anti-αGal IgE antibodies have been described to be involved in its onset, development, or progression. Anti-αGal IgE antibodies-mediated disease may include Lyme disease, the αGal syndrome or allergy to cat's dander (Gronlund H *et al.* 2009). Allergy to cat's dander may also be considered as part of the αGal syndrome where sensitization occurs further to inhalation of cat's dander, which contains αGal-glycosylated IgA. In a preferred embodiment, said disease is the αGal syndrome.

The term "avidity" as used herein may refer to the measure of the strength of binding between an antigen-binding molecule and the pertinent antigen. Avidity is related to both the affinity between an antigenic determinant and its antigen binding site on the antigen-binding molecule and the number of pertinent binding sites present on the antigen-binding molecule.

The term "recombinant protein" as used herein refers to a protein produced or expressed using a recombinant expression vector, where the expression vector comprises a nucleic acid encoding the recombinant protein, such that introduction of the expression vector into an appropriate host cell results in the production or expression of the recombinant protein. In particular embodiments, said recombinant protein is a recombinant glycoprotein. For instance, said recombinant protein or glycoprotein is a recombinant antibody. Recombinant antibodies may be chimeric or humanized antibodies, mono- or multi-specific antibodies.

The term "antibody" as used herein refers to an immunoglobulin or an antigen-binding fragment thereof. Unless otherwise specified, the term includes, but is not limited to, polyclonal, monoclonal, monospecific, polyspecific, humanized, human, single-chain, chimeric, synthetic, recombinant, hybrid, mutated, grafted, and *in vitro* generated antibodies. The antibody can include a constant region, or a portion thereof, such as the kappa, lambda, alpha, gamma, delta, epsilon, and mu constant region genes. For example, heavy chain constant regions of the various isotypes can be used, including: IgG1, IgG2, IgG3, IgG4, IgM, IgA1, IgA2, IgD, and IgE. By way of example, the light chain constant region can be kappa or lambda. Antibodies may include, but are not limited to, monoclonal antibodies (mAbs), camelid antibodies, single-chain antibodies (scFvs), Fab fragments, F(ab')2 fragments, disulphide-linked Fvs (sdFv) fragments, anti-idiotypic (anti-Id) antibodies, intra-bodies, synthetic antibodies, and epitope-binding fragments of any of the above. The term "antibody" also refers to fusion protein that includes a region equivalent to the Fc region of an immunoglobulin.

The term "antibody-containing sample" or "antibody-containing biological sample" as used herein includes biological fluids, such as whole blood, serum, plasma, synovial fluid, cerebrospinal fluid, bronchial lavage, ascites fluid, bone marrow aspirate, pleural effusion, urine, as well as any tissue or any other bodily constituent that could contain antibodies. Preferably, said "antibody-containing sample" is whole blood, serum or plasma; more preferably serum.

The term "combination" or "combination therapy" as used throughout the specification, is meant to encompass the administration of the referred therapeutic agents to a subject, in the same or separate pharmaceutical formulations, and at the same time or at different times. If the therapeutic agents are administered at different times, they should be administered sufficiently close in time to provide for the potentiating or synergistic response to occur. In such instances, it is contemplated that one would typically administer both therapeutic agents within about 12-24 hours of each other and, more preferably, within about 6-12 hours of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations. In other situations, it might be desirable to reduce the time between administration, administering both therapeutic agents within seconds or minutes to hours, preferably within about 6 hours from each other, more preferably within about 1 or 3 hours.

### Detailed description

### New glycoconjugates, compositions and methods of using thereof

In a first aspect, the present invention relates to a polymeric glycoconjugate compound comprising a terminal alpha-galactosyl moiety, wherein said compound comprises a poly-L-lysine backbone and is represented by formula (I): 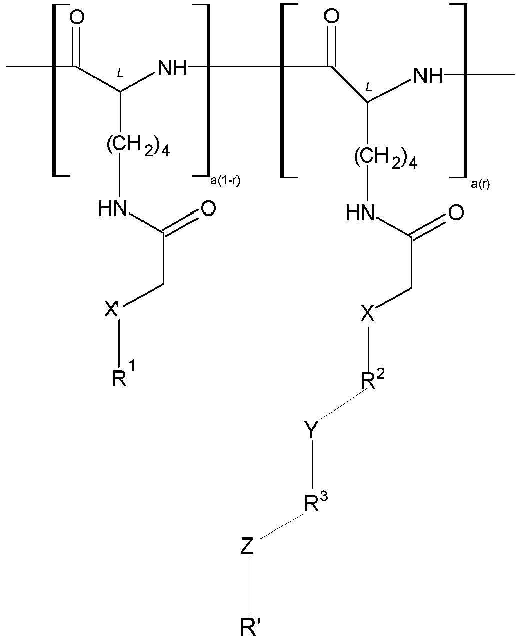
wherein R¹ is - hydrogen, alkyl, alkenyl, alkynyl, alkoxy, acyl, amine, carboxyl, carboxyl ester, cycloalkyl, cycloalkenyl, heterocycloalkyl, heterocycloalkenyl, aryl, and heteroaryl;
X, X', and Y are each independently -C(O)-, -C(O)O-, -C(O)NR-, -O-, -S-, or -NR-;
Z is a direct bond, -O-, or -C(O)NR-;
R² is -(CH₂)ₘ, -RNC(O)(CH₂)ₙC(O)NR-, -(CH₂)ₚC(O)NH(CH₂)_{q}- or -C(O)(CH₂)ₛC(O)-;
R³ is -(CH₂)ₜ-;
each occurrence of R is independently selected from the group consisting of hydrogen, OH, alkyl, alkenyl, alkynyl, alkoxy, acyl, carboxyl, carboxyl ester, cycloalkyl, cycloalkenyl, heterocycloalkyl, heterocycloalkenyl, aryl, and heteroaryl;
R' is an oligosaccharide, preferably an oligosaccharide with 2 to 6 monosaccharide units, with terminal alpha-galactosyl moiety at the non-reducing end; and
wherein m, n, p, q, s and t are each independently an integer of 1-6. Illustrative non-limitative examples are: methyl, ethyl, propyl, isopropyl, butyl, isobutyl, sec-butyl, tert-butyl, n-pentyl, neo-pentyl and n-hexyl.

The average sum of lysine units of the polylysine backbone (a) may be in the range of from 10 to 10,000, preferably from 50 to 1,500, more preferably from 250 to 1,200, from 400 to 1200, from 600 to 1200 or most preferably from 900 to 1100. For instance, (a) may be about 10, 25, 50, 75, 100, 200, 300, 400, 500, 600, 700, 800, 900, 1000, 1100 or about 1200. Polydispersity may range from 1.001 to 2.0, such as from 1.1 to 1.5, from 1.15 to 1.2. In preferred embodiments, polydispersity ranges from 1.01 to 1.2, more preferably from 1.01 to 1.1. Polydispersity index (Mw/Mn) and the degree of polymerization may be determined by Size-Exclusion Chromatography with Multi-Angle Light Scattering analysis (SEC-MALS).

The average sum of lysine units substituted with a terminal alpha-galactosyl moiety is represented as "a(r)", wherein (a) is the average number of lysine units as described herein above and (r) is the percentage of terminal alpha-galactosyl moiety expressed as decimal. In particular embodiments, (r) is more than 0.09 (preferably at least 0.12) and less than 0.23. In some embodiments of the compound of formula (I), optionally in combination of any of the above, r is from 0.12 to 0.22, more preferably from 0.15 to 0.22, even more preferably from 0.18 to 0.22. In other embodiments, optionally in combination of any of the above, r is from 0.12, 0.13 or 0.14 to less than 0.23, preferably from 0.15 to less than 0.21, more preferably from 0.18 to less than 0.21. In a preferred embodiment r is 0.18.

In particular embodiments, optionally in combination with any of the above, the compound of formula (I) is as defined herein with the proviso that said compound is not any of:
(a) a compound wherein X' is -S-, R¹ is alkoxy, X is -S-, R² is - (CH₂)ₘ-, wherein "m" is 3, Y is -C(O)NR-, wherein R is hydrogen, R³ is -(CH₂)ₜ-, wherein "t" is 3, Z is a direct bond, R' is linear B-2 trisaccharide (Galα1-3Galα1-4GlcNAc, CAS No. 101627-01-4), a is 250 and r is 0.16; or
(b) a compound wherein X' is -S-, R¹ is alkoxy, X is -S-, R² is - (CH₂)ₘ-, wherein "m" is 3, Y is -C(O)NR-, wherein R is hydrogen, R³ is -(CH₂)ₜ-, wherein "t" is 6, Z is a direct bond, R' is galactose-α-1,3-galactose, a is 1050 and r is 0.21; or
(c) a compound wherein X' is -S-, R¹ is alkoxy, X is -S-, R² is - (CH₂)ₘ-, wherein "m" is 3, Y is -C(O)NR-, wherein R is hydrogen, R³ is -(CH₂)ₜ-, wherein "t" is 3, Z is a direct bond, R' is linear B-2 trisaccharide (Galα1-3Galα1-4GlcNAc, CAS No. 101627-01-4), a is 250 and r is 0.10.

In some embodiments, optionally in combination of any of the above, X' is -C(O)-, -C(O)O-, -C(O)NR-, -O-, -S-, or -NR- and R¹ is - hydrogen, alkyl, alkenyl, alkynyl, alkoxy, acyl, amine, carboxyl or carboxyl ester. In preferred embodiments, X'-R¹ is a capping agent selected from the group consisting of thioglycerol, triethanolamine (TEA), oleic acid, thioglycolic acid, 2-mercaptoethanol, and thiolactic acid. In more preferred embodiments, X' is S and R¹ is -CH₂CH(OH)CH₂OH (i.e., the capping agent is thioglycerol).

In some embodiments, optionally in combination of any of the above, X and Y are each independently -C(O)-, -C(O)O-, -C(O)NH-, -O-, -S-, or -NH-; R² is -(CH₂)ₘ, - HNC(O)(CH₂)ₙC(O)NH-, or -(CH₂)ₚC(O)NH(CH₂)_{q}- and R³ is -(CH₂)ₜ-.

In preferred embodiments, R² is -HNC(O)(CH₂)ₙC(O)NH- and X and Y are both -NH-.
In other preferred embodiments, R² is (CH₂)₄, and X and Y are both -C(O)NH-.
In still other preferred embodiments, R² is (CH₂)₄ and S and Y are both -C(O)O-.
In other preferred embodiments, X and Y are -NR-, R2 is -RNC(O)(CH₂)ₙC(O)NR-, wherein R is hydrogen, and "n" is 4 .
In other preferred embodiments, X and Y are -NR-, R2 is -C(O)(CH₂)ₛC(O)-, wherein R is hydrogen and "s" is 4 .

In most preferred embodiments, X is -S-, R² is - (CH₂)₃- and Y is -C(O)NH- or -C(O)O-.

Typically, the oligosaccharide may consist of from 1 to 15, preferably from 1 to 10, more preferably from 1 to 6 sugar monomers selected from naturally occurring and modified sugar monomers. The skilled person is familiar with naturally occurring and modified sugar monomers and oligosaccharides comprising these sugar monomers from the standard works of organic chemistry or biochemistry, for example the Specialist Periodical Reports edited at the beginning by The Chemical Society and now by The Royal Society of Chemistry London, e.g. Ferrier et al., Carbohydrate Chemistry 29 The Royal Society of Chemistry London (1997).

Examples of sugar monomers include D- and L-aldopyranoses and D- and L-aldofuranoses, for example glyceraldehyde, erythrose, threose, ribose, arabinose, xylose, lyxose, allose, altrose, glucose, mannose, gulose, idose, galactose and talose, D- and L-ketopyranoses and D- and L-ketofuranoses, for example dihydroxyacetone, erythrulose, ribulose, xylulose, psicose, fructose, sorbose and tagatose, and also D- and L-diketopyranoses, for example pentodiulose and hexodiulose. The term sugar monomers also includes those sugar monomers which are modifications of the examples listed, for example, protected, partially protected or unprotected deoxysugars of the D- and L-configurations, preferably 2-, 3-, 4-, 5- and 6-deoxyaldoses, such as fucose, rhamnose and digitoxose, 1,2-dideoxyaldoses, such as glucal, galactal and fucal, and 1-, 3-, 4-, 5- and 6-deoxyketoses, 2-, 3-, 4-, 5- and 6-deoxyaminosugars of the D and L configurations, such as glucosamine, mannosamine, galactosamine and fucosamine, and deoxyacylaminosugars, such as N-acylglucosamine, N-acylmannosamine, N-acylgalactosamine and N-acyl-fucosamine, preferably their CrC4alkyl esters. In addition, these modifications are understood to mean aldonic, aldaric and uronic acids, such as gluconic acid or glucuronic acid, and also ascorbic acid and amino acid-carrying sugar monomers. Modified sugar monomers are also understood to mean those having a carbon chain which is longer than 6 C atoms, such as heptoses, octoses, nonoses, heptuloses, octuloses and nonuloses, and also their representatives which are substituted in accordance with the above listed criteria, such as ketodeoxyoctanic acid, ketodeoxynonanic acid, N-acylneuraminic acids and N-acylmuraminic acids.

In preferred embodiments of the compound of formula (I), optionally in combination of any of the above, said terminal alpha-galactosyl moiety is an oligosaccharide with terminal Galα1-3Gal at the non-reducing end. Preferably said oligosaccharide has 2 to 6 or 2 to 5 monosaccharide units, it may thus preferably be a disaccharide, trisaccharide, tetrasaccharide, pentasacharide or hexasaccharide.

In more preferred embodiments, said terminal alpha-galactosyl moiety is selected from the group comprising or consisting of galactose-α-1,3-galactose (Galα1-3Gal), linear B-2 trisaccharide (Galα1-3Galβ1-4GlcNAc, CAS No. 101627-01-4), linear B-6 trisaccharide (Galα1-3Galβ1-4Glc, CAS No. 56038-36-9), α1-3 galactobiosyl β-methyl glycoside; α1-3, β 1-4 galactotriose (Galα1-3Galβ1-4Gal, CAS No. 56038-36-9), galactotetraose (Galα1-3Galβ1-4Galα1-3-D-Gal, CAS No. 56038-38-1), Galili pentasaccharide (L537, Gal-α1,3Gal-β1,4GlcNAc-β1,3Gal-β1,4Glc, CAS No. 119502-59-9), Galα1-3Galβ1-3(Fucα1-4)GlcNAc (#GLY076), Galα1-3Galβ1-4(Fucα1-3)GlcNAc (#GLY075), Galα1-3[Galβ1-4GlcNAcβ1-3]4Galβ1-4Glc (#GLY079), Galα1-3[Galβ1-4GlcNAcβ1-3]3Galβ1-4Glc (#GLY078), Galα1-3Galβ1-4Glc (#GLY070), Galα1-3[Galβ1-4GlcNAcβ1-3]2Galβ1-4Glc (#GLY077), Galα1-3Galβ1-4GlcNAcβ1-3Galβ1-4Glc (#GLY071), Galα1-3Galβ1-4GlcNAc, and Galα1-3Galβ1-3GlcNAc (#GLY74-1), preferably wherein said terminal alpha-galactosyl moiety comprises or consists of terminal Galα1-3Galβ1-4GlcNAc (also referred as the Galili-epitope).

In a particularly preferred embodiment, X is -S-, R² is - (CH₂)₃-, Y is -C(O)NH-, R³ is - (CH₂)₃-, Z is a direct bond, and R' is linear B-2 trisaccharide (Galα1-3Galβ1-4GlcNAc, CAS No. 101627-01-4). In a further preferred embodiment, X' is -S-, R¹ is -CH₂CH(OH)CH₂OH, X is -S-, R² is - (CH₂)₃-, Y is -C(O)NH-, R³ is -(CH₂)₃-, Z is a direct bond, and R' is linear B-2 trisaccharide (Galα1-3Galα1-4GlcNAc, CAS No. 101627-01-4).

A method of manufacturing a compound of formula (I) has previously been described at WO9847915 or Duthaler *et al.,* 2010. In brief, this method comprises:
- Obtaining poly-l-lysine hydrobromide via ring opening polymerization of N-carboxyanhydrides using N6-TFA-l-lysine as synthetic precursor (Hadjichristidis *et al.,* 2009);
- Obtaining a protected oligosaccharide with a terminal alpha-galactosyl moiety at the non-reducing end;
- Activating the starting materials by converting poly-l-lysine hydrobromide to DMF-soluble per-chloroacetamides and by reacting the protected oligosaccharide with a spacer or linker group (e.g., converting protected αGal-epitope into thiolated αGal-epitope by reacting with γ-thiobutyrolactone), wherein the spacer or linker group serves as spacer or linker between the glycan residues and the lysine chain;
- Reacting the activated starting materials together and the remaining chloroacetamide groups are capped with a X'-R1 capping agent as described herein (e.g., thioglycerol).

In a second aspect, the invention refers to a method for obtaining a compound of formula (I). Preferably, said method comprises the steps described herein above.

In a third aspect, the invention relates to a composition comprising the polymeric glycoconjugate compound of formula (I) as described herein. Appropriate amounts of the compound of formula (I) as described herein can be formulated with pharmaceutically acceptable excipients, vehicles and/or carriers to obtain a pharmaceutical composition.

The expression "pharmaceutically acceptable" refers to molecular entities and compositions that do not produce adverse, allergic, or other untoward reactions when administered to an animal or a human. As used herein, "pharmaceutically acceptable excipients, vehicles and/or carriers" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Except insofar as any conventional media or agent is incompatible with the active ingredients of the present disclosure, its use in therapeutic compositions is contemplated. Supplementary active ingredients also can be incorporated into the compositions, provided they do not inactivate the agents according to the invention.

A composition that includes a compound of formula (I) as described herein can be delivered to a subject by a variety of routes including, without limitation, by local (e.g. topical, rectal, ocular, etc.) or systemic administration. Systemic delivery may include oral or parental (e.g., intravenous, subcutaneous, intramuscular, and intraperitoneal) administration. Additionally, it is also possible to administer the composition comprising the agent of the invention intranasally or sublingually which allows systemic administration by a non-aggressive mode of administration. Also, intraventricular administration may be adequate. A preferred route of delivery is intravascular (e.g. intraarterial or intravenous) or subcutaneous injection. In a particular embodiment, the agent for use according to the invention is administered to the subject subcutaneously or intravenously. Those skilled in the art are familiar with the principles and procedures discussed in widely known and available sources as Remington's Pharmaceutical Science (17th Ed., Mack Publishing Co., Easton, Pa., 1985) and Goodman and Gilman's The Pharmaceutical Basis of Therapeutics (8th Ed., Pergamon Press, Elmsford, N.Y., 1990) both of which are incorporated herein by reference.

The agents of the invention can be formulated as neutral or salt forms. Pharmaceutically acceptable salts include those formed with free amino groups such as those derived from hydrochloric, phosphoric, acetic, oxalic, tartaric acids and the like, and those formed with free carboxyl groups such as those derived from sodium, potassium, ammonium, calcium, ferric hydroxides, isopropylamine, thriethylamine, 2-ethylamino ethanol, histidine, procaine or similar.

Solid dosage forms for oral administration may include conventional capsules, sustained release capsules, conventional tablets, sustained-release tablets, chewable tablets, sublingual tablets, effervescent tablets, pills, suspensions, powders, granules, and gels. Formulations can include standard carriers such as pharmaceutical grades of mannitol, lactose, starch, magnesium stearate, sodium saccharide, cellulose, magnesium carbonate, etc. Such dosage forms can also comprise, as in normal practice, additional substances other than inert diluents, such as dispersing and/or granulating agents, surface active agents and/or emulsifiers, disintegrating agents, binding agents, preservatives, buffering agents, lubricating agents, and/or oils. In the case of capsules, tablets, effervescent tablets and pills, the dosage forms may also comprise buffering agents. Tablets and pills can be prepared with enteric coatings.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, and combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked polyvinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and combinations thereof.

Exemplary surface active agents and/or emulsifiers include, but are not limited to, natural emulsifiers (e.g. acacia, agar, alginic acid, sodium alginate, tragacanth, chondrux, cholesterol, xanthan, pectin, gelatin, egg yolk, casein, wool fat, cholesterol, wax, and lecithin), colloidal clays (e.g. bentonite [aluminum silicate] and Veegum [magnesium aluminum silicate]), long chain amino acid derivatives, high molecular weight alcohols (e.g. stearyl alcohol, cetyl alcohol, oleyl alcohol, triacetin monostearate, ethylene glycol distearate, glyceryl monostearate, and propylene glycol monostearate, polyvinyl alcohol), carbomers (e.g., carboxy polymethylene, polyacrylic acid, acrylic acid polymer, and carboxyvinyl polymer), carrageenan, cellulosic derivatives (e.g., carboxymethylcellulose sodium, powdered cellulose, hydroxymethyl cellulose, hydroxypropyl cellulose, hydroxypropyl methylcellulose, methylcellulose), sorbitan fatty acid esters {e.g., polyoxyethylene sorbitan monolaurate [Tween 20], polyoxyethylene sorbitan [Tween 60], polyoxyethylene sorbitan monooleate [Tween 80], sorbitan monopalmitate [Span 40], sorbitan monostearate [Span 60], sorbitan tristearate [Span 65], glyceryl monooleate, sorbitan monooleate [Span 80]), polyoxyethylene esters (e.g., polyoxyethylene monostearate [Myrj 45], polyoxyethylene hydrogenated castor oil, polyethoxylated castor oil, polyoxymethylene stearate, and Solutol), sucrose fatty acid esters, polyethylene glycol fatty acid esters (e.g., Cremophor), polyoxyethylene ethers, (e.g., polyoxyethylene lauryl ether [Brij 30]), poly(vinyl-pyrrolidone), diethylene glycol monolaurate, triethanolamine oleate, sodium oleate, potassium oleate, ethyl oleate, oleic acid, ethyl laurate, sodium lauryl sulfate, Pluronic F 68, Poloxamer 188, cetrimonium bromide, cetylpyridinium chloride, benzalkonium chloride, docusate sodium, etc. and/or combinations thereof.

Exemplary binding agents include, but are not limited to, starch (e.g., cornstarch and starch paste); gelatin; sugars (e.g., sucrose, glucose, dextrose, dextrin, molasses, lactose, lactitol, mannitol); natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone), magnesium aluminum silicate (Veegum), and larch arabogalactan); alginates; polyethylene oxide; polyethylene glycol; inorganic calcium salts; silicic acid; polymethacrylates; waxes; water; alcohol; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and sorbic acid. Exemplary alcohol preservatives include, but are not limited to, ethanol, polyethylene glycol, phenol, phenolic compounds, bisphenol, chlorobutanol, hydroxybenzoate, and phenylethyl alcohol. Exemplary acidic preservatives include, but are not limited to, vitamin A, vitamin C, vitamin E, beta-carotene, citric acid, acetic acid, dehydroacetic acid, ascorbic acid, sorbic acid, and phytic acid. Other preservatives include, but are not limited to, tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus, Phenonip, methylparaben, Germall 115, Germaben II, Neolone, Kathon, and Euxyl. In certain embodiments, the preservative is an antioxidant. In other embodiments, the preservative is a chelating agent.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and combinations thereof.

Exemplary oils include, but are not limited to, almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and combinations thereof.

Liquid dosage forms for oral administration may include emulsions, solutions, suspensions, syrups and elixirs pharmaceutically acceptable containing inert diluents commonly used in the technique, such as water. Those compositions may also comprise adjuvants such as wetting agents, emulsifying and suspending agents, and sweetening agents, flavoring and perfuming agents.

Various delivery systems are known in the art, including encapsulation in liposomes, microbubbles, emulsions, microparticles, microcapsules and the like.

Injectable preparations, for example, aqueous or oleaginous suspensions, may be formulated according with the known technique using suitable dispersing agents, wetting agents and/or suspending agents. Among the acceptable vehicles and solvents that can be used are water, Ringer's solution, and isotonic sodium chloride solution. Sterile oils are also conventionally used as solvents or suspending media.

Typically, compositions for intravenous, intramuscular, subcutaneous, intraperitoneal or intraventricular administration are solutions in sterile isotonic aqueous buffer. In some embodiments, the composition can contain minor amounts of wetting or emulsifying agents, or pH buffering agents. Illustrative non-limiting examples of pH buffering agents include Tris-HCl buffer, acetate buffer, citrate and phosphate buffer or combinations thereof. The term "acetate buffer", "citrate buffer" and "phosphate buffer" as used herein can refer to a buffer system comprising an organic acid (acetic acid, citric acid, and phosphoric acid, respectively) and a salt thereof. Each of them can be added in a sufficient amount. The pH of the composition according to the present invention may be in the range from about 4 to about 8, preferably from about 5 to about 7, including pH 5, pH 5.5, pH 6, pH 6.5 and pH 7.

Where necessary, the agent of the invention is comprised in a composition also including a solubilizing agent and a local anesthetic to ameliorate any pain at the site of the injection. Generally, the ingredients are supplied either separately or mixed together in unit dosage form, for example, as a dry lyophilized powder or water free concentrate in a hermetically sealed container such as an ampule or sachet indicating the quantity of active agent. Where the composition is to be administered by infusion, it can be dispensed with an infusion bottle containing sterile pharmaceutical grade water or saline. Where the composition is administered by injection, an ampule of sterile water for injection or saline can be provided so that the ingredients may be mixed prior to administration.

The effective quantity of the agent of the invention can vary within a wide range and, in general, will vary depending on the particular circumstances of application, duration of the exposure and other considerations. In a particular embodiment, the dose ranges from 0.01 mg/kg to 20 mg/kg, preferably from 0.05 mg/kg to 10 mg/kg, such as from 0.05 mg/kg to 2 mg/kg or 0.1 mg/kg to 5 mg/kg. In preferred embodiments, the dose ranges from 0.01 to 5 mg/Kg, preferably from 0.05 to 2.5 mg/kg and most preferably from 0.1 to 1 mg/kg. In other preferred embodiments, said dose is less than 1.0 mg/kg, such as from 0.1 to 0.5 mg/kg, including from 0.3 to 0.4 mg/kg.

The glycoconjugate compound of formula (I) or a composition comprising thereof, may be administered a single time. It typically will be administered regularly throughout the course of a treatment, for example, one, two, three, four, or more times a day, every other day, weekly, bi-weekly, every three weeks or monthly.

In a particular embodiment, optionally in combination with any of the features or embodiments described herein, a compound of formula (I) or a composition comprising thereof is administered every 12h, 24h, 48h or 72h. This administration regime may be followed during a period of 2-3 weeks, and afterwards to follow with an administration once per week, or even once every two weeks or monthly. The preferred administration regimen may be decided by the physician depending on the anti-aGal IgE antibody levels on the subject's serum.

In a fourth aspect, the present invention relates to a process of preparation of a pharmaceutical composition, said process comprising admixing the compound of formula (I) as described herein with a pharmaceutically acceptable carrier, vehicle, or excipient.

In a fifth aspect, the invention relates to a compound of formula (I) as described herein for use as a medicament.

In a sixth aspect, the invention relates to a compound of formula (I) as described herein, or a pharmaceutical composition as described herein, for use in the treatment of diseases wherein anti-aGal antibodies have been described to be involved in its onset, development, or progression, generically referred herein as a disease mediated by anti-aGal antibodies.

In a related aspect, the present application concerns a method of treating diseases wherein anti-aGal antibodies have been described to be involved in its onset, development, or progression, in a subject wherein said method comprises administering a therapeutically or prophylactically effective amount of a polymeric glycoconjugate compound of formula (I) as described herein to a subject.

In a further related aspect, the present application pertains to the use of a polymeric glycoconjugate compound of formula (I) as described herein for the manufacturing of a medicament for the treatment of diseases wherein anti-aGal antibodies have been described to be involved in its onset, development, or progression.

These diseases may be (i) autoimmune diseases, such as Graves' Disease (anti-aGal IgG; Galili U, 2013), systemic sclerosis (anti-aGal IgG; Gabrielli A, 1992); Henoch-Schonlein purpura, systemic vasculitis (anti-aGal IgA; Davin JC *et al.,* 1987) or Crohn's disease and ulcerative colitis (anti-αGal IgG; D'Alessandro M, 2002; Galili U, 2013; (ii) infectious diseases, such as infections of Gram negative bacteria (IgG, IgA, IgM; WO2016026981); Chagas' disease (IgG, IgA, IgM; Galili U, 2013) and Lyme disease (anti-αGal IgE; Tjernberg I *et al.,* 2017); or an anti-αGal IgE-mediated allergic response as described herein below.

Graves' disease is an autoimmune disease that leads to a generalized overactivity of the entire thyroid gland (hyperthyroidism). Aberrant expression of α-gal epitopes in human tissues may result in anti-αGal mediated autoimmune processes. Patients with Graves' disease display elevated titers of anti-αGal IgG, raising the possibility that α-gal epitopes aberrantly expressed on thyrocytes induce activation of quiescent anti-αGal B cells. Anti-αGal was further found to mimic thyroid-stimulating hormone (TSH) function if α-gal epitopes are expressed on TSH receptors (TSHR) (Galili U, 2013).

Chagas disease is a tropical infectious disease caused by the protozoan parasite *Trypanosoma cruzi,* which is transmitted to animals and people by insect vectors. The infective trypomastigote forms of the parasite can invade several human blood cell populations, including monocytes and dendritic cells. *T. cruzi* express on cell membrane multiple α-gal epitopes on glycoinositolphospholipids and lipophosphoglycans. Intracellular parasites produce and release glycoinositolphospholipids and lipophosphoglycans with α-gal epitopes. These released glycoconjugates stimulate the immune system for continuous production of anti-aGal at high titers. Chagas' disease caused by *Trypanosoma cruzi* is marked by 10-30-fold higher anti-aGal titers than in uninfected individuals. Anti-αGal further interacts with these glycoconjugates resulting in extensive localized 'autoimmune-like' inflammatory reactions characterized by extensive infiltration of macrophages and lymphocytes and which result in cardiomyopathy, hepatomegaly and megacolon characteristic of acute and chronic Chagas' disease (Galili U, 2013).

Lyme borreliosis (LB) is an infectious disease caused by bacteria of the genus Borrelia (e.g: *B. burgdorferi),* which are transmitted by hard ticks of the genus Ixodes. LB is the most known tick-borne infection in both Europe and North America (Tjernberg I *et al.,* 2017). The most frequent clinical manifestation is erythema migrans (EM) (frequency of at least 70% of all clinical LB), an expanding skin redness that usually develops at the site of a tick bite (Stanek G *et al.,* 2018). Early disseminated infection particularly causes multiple EM or neurologic disease, and late manifestations predominantly include arthritis in North America, and acrodermatitis chronica atrophicans (ACA) in Europe (Kullberg BJ *et al.,* 2020). IgE anti-α-Gal is commonly found in an LB endemic area with a frequency of 10-22% with a male predominance. Furthermore, the level of IgE anti-α-Gal in α-Gal positive cases decreases over time in EM patients (Tjernberg I *et al.,* 2017).

The compound of formula (I) as described herein can be used alone or in combination with one or more additional therapeutic agents. This may be part of the same or separate compositions.

### Glycoconjugates for use in the treatment of anti-αGal IgE antibodies-mediated diseases

In an additional aspect, the application relates to a polymeric glycoconjugate compound comprising a terminal alpha-galactosyl moiety for use in a method of treating anti-αGal IgE antibodies-mediated diseases in a subject.

Preferably, said compound comprises a poly-L-lysine backbone and is represented by formula (I): 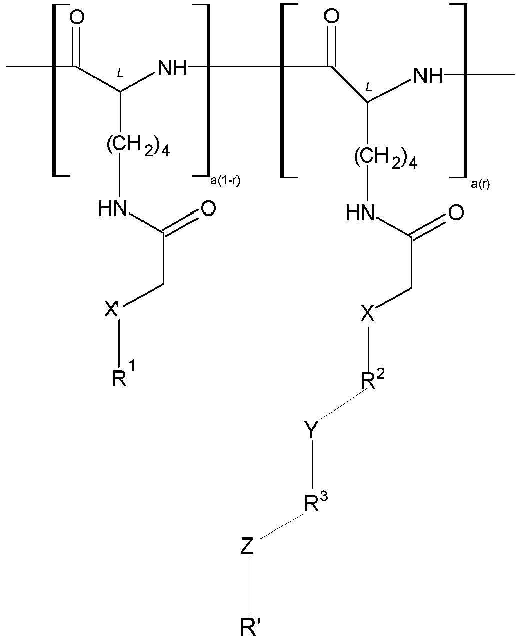
wherein R¹ is - hydrogen, alkyl, alkenyl, alkynyl, alkoxy, acyl, amine, carboxyl, carboxyl ester, cycloalkyl, cycloalkenyl, heterocycloalkyl, heterocycloalkenyl, aryl, and heteroaryl;
X, X', and Y are each independently -C(O)-, -C(O)O-, -C(O)NR-, -O-, -S-, or -NR-;
Z is a direct bond, -O-, or -C(O)NR-;
R² is -(CH₂)ₘ, -RNC(O)(CH₂)ₙC(O)NR-, -(CH₂)ₚC(O)NH(CH₂)_{q}- or -C(O)(CH₂)ₛC(O)-;
R³ is -(CH₂)ₜ-;
each occurrence of R is independently selected from the group consisting of hydrogen, OH, alkyl, alkenyl, alkynyl, alkoxy, acyl, carboxyl, carboxyl ester, cycloalkyl, cycloalkenyl, heterocycloalkyl, heterocycloalkenyl, aryl, and heteroaryl;
R' is an oligosaccharide, preferably an oligosaccharide with 2 to 6 monosaccharide units, with terminal alpha-galactosyl moiety at the non-reducing end; and
wherein m, n, p, q, s and t are each independently an integer of 1-6.

The average sum of lysine units of the polylysine backbone (a) may be in the range of from 10 to 10,000, preferably from 50 to 1,500, more preferably from 250 to 1,200, from 400 to 1200, from 600 to 1200 or most preferably from 900 to 1100. For instance, (a) may be about 10, 25, 50, 75, 100, 200, 300, 400, 500, 600, 700, 800, 900, 1000, 1100 or about 1200.

Polydispersity may range from 1.001 to 2.0, such as from 1.1 to 1.5, from 1.15 to 1.2. In preferred embodiments, polydispersity ranges from 1.01 to 1.1.

The average sum of lysine units substituted with a terminal alpha-galactosyl moiety is represented as "a(r)", wherein (a) is the number of lysine units as described herein and (r) is the percentage of terminal alpha-galactosyl moiety expressed as decimal. In particular embodiments, (r) is from more than 0.09 (preferably at least 0.12) to 1, including up to 0.9, 0.8, 0.7, 0.6, 0.5 or up to 0.4. In some embodiments of the compound of formula (I), optionally in combination of any of the above, r is from 0.1 to 0.3; preferably from 0.12 to 0.29, more preferably from 0.15 to 0.28, even more preferably from 0.18 to 0.27. In a particular embodiment r is 0.12, 0.18 or 0.27.

In a preferred embodiment of the compound of formula (I), r is from 0.15 to 0.28 or from 0.18 to 0.27 and "a" is from 900 to 1100. In another preferred embodiment, r is from 0.18 to 0.27 and "a" is from 600 to 1200 or from 900 to 1100.

Other features and embodiments of the glycoconjugates and compositions comprising thereof for use in a method of treating anti-αGal IgE antibodies-mediated diseases in a subject according to the invention are as described herein above under the first and second aspects of the invention.

In a related aspect, the present application relates to a method of treating anti-αGal IgE antibodies-mediated diseases in a subject wherein said method comprises administering a therapeutically or prophylactically effective amount of a polymeric glycoconjugate compound, preferably of formula (I) as described herein, to a subject.

In a further related aspect, the present application pertains to the use of a polymeric glycoconjugate compound, preferably of formula (I) as described herein, for the manufacturing of a medicament for the treatment of anti-aGal IgE antibodies-mediated diseases.

In preferred embodiments of any of the above, said disease mediated by anti-αGal IgE antibodies is an allergic response in a subject sensitized to αGal. Such an allergic response may also be referred as the αGal syndrome. The αGal syndrome is an IgE-mediated allergy to the disaccharide galactose-α-1,3-galactose (αGal).

Sensitization to αGal has been described to occur further to parenteral exposure (e.g., subcutaneous) to proteins with glycosylation patterns containing the αGal epitope. Tick bites are assumed to be the most frequent and most important primary sensitization source to α-Gal In particular, hard ticks of the genus *Ixodes* and *Amblyomma,* like *Amblyomma americanum* (North America), *Ixodes ricinus* (Europe), *Ixodes holocyclus* (Australia), *Amblyomma testudinarium* (Japan) and members of the *Amblyomma cajennense* complex, such as *Amblyomma sculptum* (Panama) have been associated with αGal-IgE sensitization (Berg EA *et al.,* 2014; Wilson JM *et al.* 2017). Moreover, it remains likely that more species of ticks, and even other ectoparasites such as chiggers, are causally related to αGal-sensitization (Wilson JM and Platts-Mills TAE, 2019). Araujo RN *et al.* showed that subcutaneous injection of tick saliva was sufficient to induce α-Gal-specific IgE in an α-Gal knockout mouse model (Araujo RN *et al.,* 2016).

In some embodiments, said subject has been sensitized to αGal further to a hard tick (Family Ixodidae) bite, more specifically by the saliva injected by a hard tick bite. This hard tick is preferably of the genera *Amblyomma* or *Ixodes,* more preferably selected from *Amblyomma americanum, Ixodes ricinus, Ixodes holocyclus, Amblyomma testudinarium* and members of the *Amblyomma cajennense* complex, such as *Amblyomma sculptum.*

Sensitization to αGal has also been described to occur further to inhalation of cat's dander, which contains αGal-glycosylated IgA.

Said αGal sensitized subject is characterized by an increase of anti-aGal IgE antibody levels (e.g., serological levels) with respect to a non-sensitized subject. The anti-αGal IgE antibodies may be total or normalized (e.g. by total antibody content). These also may be expressed as weight/volume (e.g. mg/ml) or international units (IU)/volume (e.g. IU/ml). The levels of a non-sensitized subject are considered as a reference value.

The term "reference value", as used herein, relates to a predetermined criterium used as a reference for evaluating the values or data obtained from the samples collected from a subject. This "reference value" may also be referred as "cut-off value" or "threshold value".

The term "reference value" may refer to a value determined in one or more samples, generally mean values of preferably a statistically significant number of samples, obtained from a reference or control population (i.e. a population of non-sensitized subjects). Determination of the "reference value" is carried out under similar or identical conditions as the biological sample of the tested subject. Typically, said reference samples are from the same type of biological sample, e.g., the same tissue or cell type and have undergone the same procedures for their obtaining and preservation. Alternatively, said "reference values" are pre-established values, generally mean values, in the control or reference population.

The term "increased" as used herein refers to an anti-αGal IgE antibody levels value which is higher than a reference value. Preferably, this value is considered to be higher than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than a reference value.

Alternatively, or in addition, subjects sensitized to αGal have more than about 1.1,1.2, 1.3, 1.4, 1.5, 2, 3, 4, 5, 6, 7, 8, 9, 10, 15, 20, 50, 100 or more fold levels increase than the reference value as described herein.

As it can be seen in Figure 3 the serological levels anti-aGal IgE antibodies in a non-sensitized subject (CN) are about 0 when expressed as normalized optical density units. Thus, an αGal sensitized subject may also be characterized by the presence of anti-aGal IgE antibodies in an antibody-containing biological sample (e.g. serum). In preferred embodiments, an αGal sensitized subject is characterized by having anti-αGal IgE antibody levels in serum of at least 2 IU/mL or more than 2% of the total IgE (Platts-Mills TAE *et al.,* 2019).

Said increase of anti-αGal IgE antibodies is generally associated to total IgE increase with respect to a non-sensitized subject. Furthermore, said increase of anti-αGal IgE may also be associated to an increase of anti-αGal IgG antibodies (in particular, IgG1 and IgG3 isotypes) with respect to a non-sensitized subject (see Example 3 and Rispens T *et al.* 2013).

In some embodiments, instead of determining the levels of anti-αGal IgE antibodies in a subject, the levels of IgE against cat IgA antibodies may alternatively be determined (Gronlund H *et al.* 2009).

Methods for the detection or determination of levels of antibodies (e.g., anti-IgE antibodies) in an antibody-containing sample can be conducted by any method known in the art. Traditionally, quantification of proteins in solution has been carried out by immunoassays on a solid support. Said immunoassay may be for example an enzyme-linked immunosorbent assay (ELISA), a fluorescent immunosorbent assay (FIA), a chemiluminescence immunoassay (CIA), or a radioimmunoassay (RIA), an immunoradiometric assay (IRMA), an enzyme multiplied immunoassay, a solid-phase radioimmunoassay (SPROA), a fluorescence polarization (FP) assay, a fluorescence resonance energy transfer (FRET) assay, a time-resolved fluorescence resonance energy transfer (TR-FRET) assay, a surface plasmon resonance (SPR) assay, magnetic beads-based immunoassay (MBI), an electrochemiluminescent immunoassay (ECL). Multiplex and any next generation versions of any of the above, such as bead-based flow-cytometry immunoassays (e.g., based on the Luminex xMAP technology) are specifically encompassed. In a particular embodiment, said immunoassay is an ELISA assay or any multiplex version thereof.

Other methods that can be used for quantification of proteins in solution are techniques based on mass spectrometry (MS) such as liquid chromatography coupled to mass spectrometry (LC / MS), described for example in US2010/0173786, or tandem LC-MS / MS (WO2012/155019, US2011/0039287) and multiplex versions of the above techniques, as well as the next generation of such techniques and combinations thereof.

In other embodiments, determination of whether anti- anti-αGal IgE levels are increased with respect to a reference value is conducted by lateral flow tests, also known as Lateral Flow Immunochromatography (LFIC) tests or just Lateral Flow Immunoassays (LFI). These are simple small devices intended to detect a target analyte in a given sample. These tests are simple, fast, and cheap and do not require specialized and costly equipment nor qualified personnel. Although there are several commercially available semi-quantitative tests, LFIC tests are mainly qualitative tests and they just give a positive or negative result based on the presence or absence of the target analyte at a specific concentration.

In a particular embodiment, anti-αGal IgE antibody levels present in an antibody-containing sample are determined by an immunoassay wherein an oligosaccharide comprising or consisting of an (terminal) αGal epitope (Galα1-3Gal) as described herein, has been coated on a solid-phase support. Below it is generally described a method for the determination of anti-αGal antibodies of a selected isotype.

An oligosaccharide or polymeric structure containing a terminal αGal epitope may be bound to a solid-phase support by adsorption or covalent binding via an amide or ester or disulfide bonds. Coating antigens can also be bound to a solid support using binding pairs such as biotin and avidin or antibody and antigen. Any means known in the art for immobilizing a polymer (e.g., a protein or polypeptide or polyacrylamide) or oligosaccharide to a solid support can be used. After said oligosaccharide or polymer is coated, the solid-phase support can be incubated with a blocking solution (containing a blocking protein such as bovine serum albumin) to reduce non-specific adsorption of antibodies in a test sample to the support surface.

Various solid-phase supports can be used, including but not limited to glass, polystyrene, polypropylene, methacrylate, nitrocellulose, agarose, dextran or other materials. Suitable forms of the solid-phase supports can include beads, microparticles, columns, tubes, fabrics or plates formed from or coated with these materials. In a preferred embodiment the solid support comprises microtiter wells, such as a 96-well microtiter plate.

A detection antibody is used to assess the anti-αGal antibodies bound to the terminal αGal antigen previously coated to the solid-phase support. The detection antibody used for this purpose can be isotype-specific, e.g. IgA, IgD, IgE, IgG, or IgM. The isotype-specific detection antibody can be labeled and detected. Antibody subclasses (e.g., IgG1, IgG2, IgG3, IgG4) can also be determined.

A label can be any composition which is detectable. Any analytical means known in the art can be used for determining or detecting the detection antibody. This include the use of spectroscopy, chemistry, photochemistry, biochemistry, immunochemistry, or optics. The label can be, for example, an enzyme (e.g., horseradish peroxidase (HRP), alkaline phosphatase, beta-galactosidase, and others commonly used in an ELISA), a radiolabel (e.g., ³H, ¹²⁵I, ³⁵S, ¹⁴C, or ³²P), a chemiluminescent compound (e.g. luciferin, and 2,3-dihydrophthalazinediones, luminol, etc.), a fluorescent dye (e.g., fluorescein isothiocyanate, Texas red, rhodamine, etc.), or any other dye known in the art.

The label may be coupled directly (covalent binding) or indirectly (e.g., via binding pairs such as biotin and avidin) to the detection antibody according to methods well known in the art. As indicated above, a wide variety of labels may be used. The choice of label may depend on sensitivity required, ease of conjugation with the compound, stability requirements, available instrumentation, or disposal provisions.

Detection antibodies can be detected or quantified by any suitable method known in the art. A label on an antibody can be detected by a gamma counter if the label is a radioactive gamma emitter, or by a fluorimeter, if the label is a fluorescent material. In a preferred embodiment, the detection antibody is detected using an HRP-conjugated species-specific immunoglobulin. Any known substrate of HRP can be used. Non-limiting examples are ABTS (2,2'-Azinobis [3-ethylbenzothiazoline-6-sulfonic acid]-diammonium salt), OPD (o-phenylenediamine dihydrochloride) and TMB (3,3',5,5'-tetramethylbenzidine). Each of these substrates yields a reaction product that can be detected optically with a maximum absorbance at 410 nm and 650 nm, at 492 nm and at 450 nm, respectively.

Results of the assay may be qualitative or quantitative. The amount of label associated with the support can be compared with positive and negative controls in order to determine the presence of anti-aGal antibodies. The controls are typically run concomitantly with the sample to be tested. A positive control can be a serum containing antibodies that are immunoreactive to the αGal epitope. A negative control can be a serum that does not contain antibodies that are immunoreactive to the αGal epitope. For quantitation, a standard curve using known quantities of pure isotype of immunoglobulins or anti-αGal antibodies can be generated and/or used.

Antibodies for use as positive controls may be produced using glycoconjugates comprising a terminal alpha-galactosyl moiety which comprises or consists of the αGal epitope. Preferred glycoconjugates are as described herein. Any type of antibody known in the art can be generated to bind specifically to the αGal epitope. Monoclonal or polyclonal antibodies can be made as is well known in the art.

Any technique for purifying antibodies available in the art can be used. For example, antibodies can be purified by known methods such as affinity separation using protein A, high pressure liquid chromatography on reverse phase alkylated silica gel, or gel filtration. Antibodies can also be passed over a solid phase to which the oligosaccharide comprising or consisting of an (terminal) αGal epitope is bound. The anti-aGal antibodies will bind to the oligosaccharide comprising or consisting of an (terminal) αGal epitope bound to the solid support and the contaminants can be washed away. The bound antibodies can be eluted, for example, by a drastic pH change (under acid or alkaline conditions) or with a buffer having a high salt concentration to diminish affinity interaction Ab-Ag.

The particular parameters employed in the assay for anti- αGal antibody levels determination can vary widely depending on various factors such as the concentration of antibody in the sample, the nature of the sample, the type of immunoassay employed. Optimal conditions can be readily established by those of ordinary skill in the art. Typical assay conditions include a temperature range of about 4°C to about 45°C, preferably around 20°C, and a pH value range of about 5 to 9. Incubation times can also vary widely depending upon the nature of the assay, and generally range from about 0.1 minute to about 24 hours. A broad variety of buffers, for example TRIS-buffered saline, may be employed, and other reagents such as salt to enhance ionic strength, proteins such as serum albumin, stabilizers, and non-ionic detergents may also be included. Exemplary conditions for determining total immunoglobulins and anti-αGal antibodies of the IgE, IgG, IgM and IgA isotypes are given in Example 1.

Anaphylactic reactions mediated by anti-aGal immunoglobulins of the IgE isotype have been described to occur in sensitized subjects further to a second or further exposure to protein with a glycosylation pattern containing the αGal epitope by the oral or parenteral route. By the oral route the onset of symptoms has been described to be delayed (> 2 hours after ingestion) and may occur further to the ingestion of non-primate mammalian derived-products, such as non-primate mammalian (e.g. beef or pork) muscle meat and innards (e.g. kidney), but also dairy products, gelatin in sweets or porcine pancreatic enzymes in digestion aids. Exposure by the parenteral route is typically characterized by a rapid onset of symptoms (< 30 minutes since exposure) and may occur further to the administration of drugs derived from non-primate mammalian cells and tissue, like recombinant glycoproteins (e.g., cetuximab), antivenom, gelatin in colloids or vaccines. Typically, parenteral exposure occurs further to intravenous, intramuscular or subcutaneous administration. In some instances, parenteral exposure may be via inhalation such as described for allergy to cat's dander containing αGal-glycosylated IgA (Gronlund H *et al.,* 2009) or as described for cattle workers assisting during calving further to inhalation of amniotic fluid (Hilger C *et al.,* 2019).

In addition to the onset of anaphylactic reactions, the anti-αGal IgE antibodies-mediated response characterizing the αGal syndrome may be involved in xeno-rejection and deterioration of biological heart valves (Hilger C *et al.,* 2019).

In a particular embodiment, the allergic reaction mediated by anti-αGal immunoglobulins of the IgE isotype occurs in the sensitized subjects further to exposure to a protein with a glycosylation pattern containing the αGal epitope by the parenteral route (e.g. further to intravenous, intramuscular or subcutaneous injection or further to inhalation). In preferred embodiments, said protein is a recombinant protein produced in non-primate mammalian organisms or cells. Examples of proteins which have been described to have the αGal epitope in its glycosylation pattern are bovine serum albumin (BSA) and bovine thyroglobulin (Homann A *et al.,* 2017).

Mammalian cell systems for protein production include a variety of transformed and/or genetically modified cell lines. Said non-primate mammalian cells may be for instance dog or rodent cells. Examples of dog cells are MDCK cells (ATCC CCL-34). Examples of rodent cells are hamster cells, such as BHK21-F, HKCC cells, or CHO cells; and mouse cells, such as SP2/0. Suitable expression systems also include transgenic animals described in Gene Expression Systems, Academic Press, eds. Fernandez et al., 1999.

Depending on the recombinant protein being produced the type and extent of glycosylation will vary and consequently the percentage of each oligosaccharide structures. In a further preferred embodiment, said protein is an antibody. Antibodies are glycosylated at conserved, N-linked glycosylation sites in the Fc regions of immunoglobulin heavy chains. Moreover, each antibody isotype has a distinct variety of N-linked carbohydrate structures in the constant regions. For example, each heavy chain of an IgG antibody has a single N-linked glycosylation site at Asn297 of the CH2 domain (Jefferis J, 2009). IgA antibodies have N-linked glycosylation sites within the CH2 and CH3 domains, IgE antibodies have N-linked glycosylation sites within the CH3 domain, and IgM antibodies have N-linked glycosylation sites within the CH1, CH2, CH3, and CH4 domains (see Arnold JN *et al.,* 2005; Mattu TS *et al.,* 1998; Nettleton MY *et al.,* 1995). In addition, glycosylation sites may be present at the variable region.

Cetuximab is a chimeric IgG1 monoclonal antibody produced in a mouse cell line (SP2/0). It has been reported the presence of bi-and tri-antennary glycans with terminal α-Gal epitopes on the variable region of the heavy chain (VH). Glycans are also common on the constant heavy chain domain (CH2) but these only rarely have terminal α-Gal epitopes. Oligosaccharides present in the Fc domain of IgG attach at Asn297, and are mainly of the complex diantennary type and are comprised of a core heptasaccharide (GlcNac₂Man₃GlcNac₂) with variable addition of fucose, galactose, bisecting N-acetylglucosamine and sialic acid. Figure 13 (Figure 3, adapted from Wilson JM and Platts-Mills TAE, 2018) shows the structure of cetuximab and its glycosylation profile.

In a particular embodiment, said antibody is cetuximab. Other antibodies which have been described to have a glycosylation pattern which may contain terminal αGal epitopes include but are not limited to adalimumab and infliximab (Homann A *et al.* 2017).

In some embodiments, optionally in combination with one or more of the embodiments described herein, further to administration of the compound of formula (I), said compound removes at least 70%, preferably at least 80%, more preferably at least 90% or at least 95% of the serum anti-αGal immunoglobulins of the IgE isotype. Reference is made to Example 3, wherein is shown that treatment with RA0118 as described in Fig. 2 (Group 3) achieved an *in vivo* removal of more than 70% of the anti-αGal IgE antibodies in the serum of αGal sensitized GalT-KO mice (Figure 8).

In some embodiments, optionally in combination with one or more of the embodiments described herein, a compound of formula (I) is characterized by inhibiting or removing at least 70%, preferably at least 80%, more preferably at least 90% or at least 95% of the anti-αGal antibodies of the IgE isotype from a serum containing anti-αGal antibodies at a concentration of 0.005 mg/mL to 0.1 mg/ml as *in vitro* determined by any method as described herein, such as by ELISA determination. In preferred embodiments, said degree of inhibition is obtained at a concentration of 0.005 mg/mL to 0.05 mg/mL, preferably of 0.005 mg/mL to 0.02 mg/mL, more preferably of 0.005 mg/mL to 0.01 mg/mL, even more preferably at about 0.005 mg/mL.

In preferred embodiments, the compound of formula (I) is administered parenterally. Preferably, by intravenous, intramuscular, or subcutaneous administration, more preferably by subcutaneous administration. Preferred features and embodiments of pharmaceutical compositions and dose and administration schedules are as described herein above.

The compound of formula (I) as described herein can be used alone or in combination with one or more additional therapeutic agents. This may be part of the same or separate compositions. In preferred embodiments, the compound of formula (I) for use in a method of treating anti-aGal IgE antibodies-mediated diseases in a subject is used in combination with one or more agents used in the treatment of allergic conditions. As illustrative examples, these may include one or more of the following and combinations thereof:
- Antihistamines: Cetirizine (Zyrtec), fexofenadine (Allegra), levocetirizine (Xyzal), loratadine (Claritin, Alavert), Brompheniramine (Dimetapp allergy, Nasahist B), chlorpheniramine (Chlor-Trimeton), clemastine (Tavist), diphenhydramine (Benadryl), Desloratadine (Clarinex) and Azelastine (Astelin).
- Leukotriene modifiers (leukotriene antagonists): zafirlukast (Accolate), montelukast (Singulair) and zileuton (Zyflo).
- Mast cell inhibitors: Cromolyn sodium (Nasalcrom).
- Steroids: Flonase, Nasacort Allergy 24HR, Rhinocort, Beconase, Dymista, Nasarel, Nasonex, Qnasl, Vancenase, Veramyst, and Zetonna.
- Epinephrine (adrenaline).
- Anticholinergics: Ipratropium bromide (Atrovent).
- Antibodies directed to IgE: Omalizumab (Xolair).

It is contemplated that any features described herein can optionally be combined with any of the embodiments of any compound, composition, pharmaceutical composition, kit, medical use, method of treatment, method of manufacturing a medicament and combination therapies of the invention; and any embodiment discussed in this specification can be implemented with respect to any of these. It will be understood that particular embodiments described herein are shown by way of illustration and not as limitations of the invention.

All publications and patent applications are herein incorporated by reference to the same extent as if each individual publication or patent application was specifically and individually indicated to be incorporated by reference.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of' and "consists essentially of'. As used herein, the phrase "consisting essentially of' limits the scope of a claim to the specified materials or steps and those that do not materially affect the basic and novel characteristic(s) of the claimed invention. As used herein, the phrase "consisting of' excludes any element, step, or ingredient not specified in the claim except for, e.g., impurities ordinarily associated with the element or limitation.

The term "or combinations thereof' as used herein refers to all permutations and combinations of the listed items preceding the term. For example, "A, B, C, or combinations thereof' is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

For reasons of completeness, various aspects of the invention are set out in the following numbered clauses:

### CLAUSES

1. A polymeric glycoconjugate compound comprising a terminal alpha-galactosyl moiety, wherein said compound comprises a poly-L-lysine backbone and is represented by formula (I): 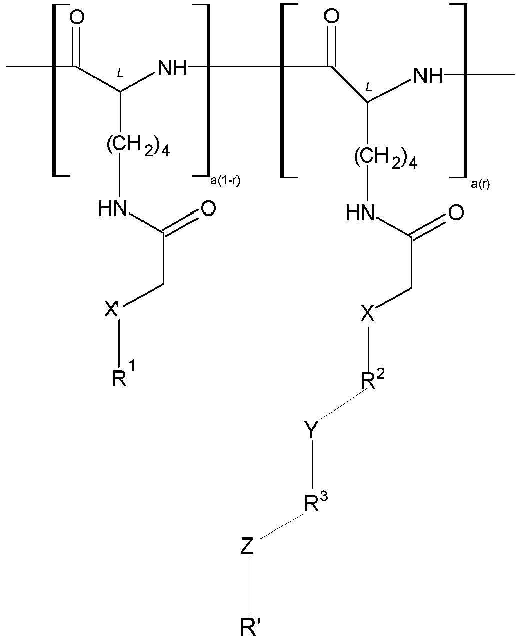
   wherein R¹ is - hydrogen, alkyl, alkenyl, alkynyl, alkoxy, acyl, amine, carboxyl, carboxyl ester, cycloalkyl, cycloalkenyl, heterocycloalkyl, heterocycloalkenyl, aryl, and heteroaryl;
   X, X', and Y are each independently -C(O)-, -C(O)O-, -C(O)NR-, -O-, -S-, or -NR-;
   Z is a direct bond, -O-, or -C(O)NR-;
   R² is -(CH₂)ₘ, -RNC(O)(CH₂)ₙC(O)NR-, -(CH₂)ₚC(O)NH(CH₂)_{q}- or -C(O)(CH₂)ₛC(O)-;
   R³ is -(CH₂)ₜ-;
   each occurrence of R is independently selected from the group consisting of hydrogen, OH, alkyl, alkenyl, alkynyl, alkoxy, acyl, carboxyl, carboxyl ester, cycloalkyl, cycloalkenyl, heterocycloalkyl, heterocycloalkenyl, aryl, and heteroaryl;
   R' is an oligosaccharide, preferably an oligosaccharide with 2 to 6 monosaccharide units, with terminal α-galactosyl moiety at the non-reducing end;
   wherein m, n, p, q, s, and t are each independently an integer of 1-6;
   and wherein
   a is from 50 to 1200; and r is from 0.12 to less than 0.23;
   with the proviso that said compound is not any of:
      - a compound wherein X' is -S-, R¹ is alkoxy, X is -S-, R² is - (CH₂)ₘ-, wherein "m" is 3, Y is -C(O)NR-, wherein R is hydrogen, R³ is -(CH₂)ₜ-, wherein "t" is 3, Z is a direct bond, R' is linear B-2 trisaccharide (Galα1-3Galβ1-4GlcNAc, CAS No. 101627-01-4), a is 250 and r is 0.16; or
      - a compound wherein X' is -S-, R¹ is alkoxy, X is -S-, R² is - (CH₂)ₘ-, wherein "m" is 3, Y is -C(O)NR-, wherein R is hydrogen, R³ is -(CH₂)ₜ-, wherein "t" is 6, Z is a direct bond, R' is galactose-α-1,3-galactose, a is 1050 and r is 0.21.
2. The polymeric glycoconjugate compound according to clause 1, wherein r is from 0.12 to 0.22, preferably from 0.15 to less than 0.21, more preferably from 0.18 to less than 0.21.
3. The polymeric glycoconjugate compound according to any of clauses 1 or 2, for use as a medicament.
4. A composition comprising the polymeric glycoconjugate compound according to any of clauses 1 or 2.
5. The composition according to clause 4, wherein said composition is a pharmaceutical composition further comprising a pharmaceutically acceptable excipient, carrier or vehicle.
6. The polymeric glycoconjugate compound according to any of clauses 1 or 2, or the composition according to clause 5, for use in the treatment of a disease mediated by anti-αGal antibodies.
7. A polymeric glycoconjugate compound comprising a terminal alpha-galactosyl moiety; wherein said compound comprises a poly-L-lysine backbone and is represented by formula (I): 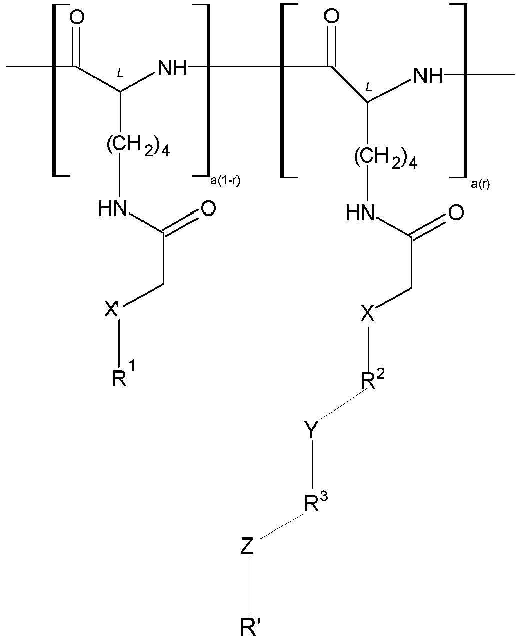
   wherein R¹ is hydrogen, alkyl, alkenyl, alkynyl, alkoxy, acyl, amine, carboxyl, carboxyl ester, cycloalkyl, cycloalkenyl, heterocycloalkyl, heterocycloalkenyl, aryl, and heteroaryl; X, X' and Y are each independently -C(O)-, -C(O)O-, -C(O)NR-, -O-, -S-, or -NR-;
      Z is a direct bond, -O-, or -C(O)NR-;
   R² is -(CH₂)ₘ, -RNC(O)(CH₂)ₙC(O)NR-, -(CH₂)ₚC(O)NH(CH₂)_{q}- or -C(O)(CH₂)ₛC(O)-;
      R³ is -(CH₂)ₜ-;
      each occurrence of R is independently selected from the group consisting of hydrogen, OH, alkyl, alkenyl, alkynyl, alkoxy, acyl, carboxyl, carboxyl ester, cycloalkyl, cycloalkenyl, heterocycloalkyl, heterocycloalkenyl, aryl, and heteroaryl;
      R' is an oligosaccharide, preferably an oligosaccharide with 2 to 6 monosaccharide units, with terminal α-galactosyl moiety at the non-reducing end;
      wherein m, n, p, q, s and t are each independently an integer of 1-6;
   wherein
   a is from 50 to 1200; and r is from 0.12 to 1; and
   wherein said compound is for use in a method of treating a disease, disorder or condition where anti-aGal IgE antibodies have been described to be involved in its onset, development, or progression (an anti-αGal IgE antibodies-mediated disease) in a subject.
8. The polymeric glycoconjugate compound for use according to clause 7, wherein r is from 0.12 to 0.3; preferably from 0.12 to 0.29, more preferably from 0.15 to 0.28, even more preferably from 0.18 to 0.27.
9. The polymeric glycoconjugate compound according to any of clauses 1 or 2, or the polymeric glycoconjugate compound for use according to any of clauses 7 or 8, wherein "a" is from 160 to 1200, preferably from 400 to 1200, more preferably from 600 to 1200, even more preferably from 900 to 1100.
10. The polymeric glycoconjugate compound according to any of clauses 1 or 2, or the polymeric glycoconjugate compound for use according to any of clauses 7 to 9, wherein X is -S-, R² is - (CH₂)₃-, Y is -C(O)NH- or -C(O)O-.
11. The polymeric glycoconjugate compound according to any of clauses 1 or 2, or the polymeric glycoconjugate compound for use according to any of clauses 7 to 10, wherein said terminal alpha-galactosyl moiety is selected from the group comprising or consisting of galactose-α-1,3-galactose (Galα1-3Gal), linear B-2 trisaccharide (Galα1-3Galβ1-4GlcNAc, CAS No. 101627-01-4), linear B-6 trisaccharide (Galα1-3Galβ1-4Glc, CAS No. 56038-36-9), α1-3 galactobiosyl β-methyl glycoside; α1-3, β1-4 galactotriose (Galα1-3Galβ1-4Gal, CAS No. 56038-36-9), galactotetraose (Galα1-3Galβ1-4Galα1-3-D-Gal, CAS No. 56038-38-1), Galili pentasaccharide (L537, Gal-α1,3 Gal-β1,4GlcNAc-β1,3Gal-β1,4Glc, CAS No. 119502-59-9), Galα1-3Galβ1-3(Fucα1-4)GlcNAc (#GLY076), Galα1-3Galβ1-4(Fucα1-3)GlcNAc (#GLY075), Galα1-3[Galβ1-4GlcNAcβ1-3]4Galβ1-4Glc (#GLY079), Galα1-3[Galβ1-4GlcNAcβ1-3]3Galβ1-4Glc (#GLY078), Galα1-3[Galβ1-4Glc (#GLY070), Galα1-3[Galβ1-4GlcNAcβ1-3]4Galβ1-4Glc (#GLY077), Galα1-3Galβ1-4GlcNAcβ1-3Galβ1-4Glc (#GLY071), Galα1-3Galβ1-4GlcNAc, and Galα1-3Galβ1-3GlcNAc (#GLY74-1), preferably wherein said terminal alpha-galactosyl moiety comprises or consists of terminal Galα1-3Galβ1-4GlcNAc.
12. The polymeric glycoconjugate compound according to any of clauses 1 or 2, or the polymeric glycoconjugate compound for use according to any of clauses 7 to 11, wherein X' is -S-, R¹ is -CH₂CH(OH)CH₂OH, X is -S-, R² is - (CH₂)₃-, Y is -C(O)NH-, R³ is - (CH₂)₃-, Z is a direct bond, and R' is linear B-2 trisaccharide (Galα1-3Galβ1-4GlcNAc, CAS No. 101627-01-4).
13. The polymeric glycoconjugate compound for use in a method according to any of clauses 7 to 12, wherein said disease mediated by anti-aGal IgE antibodies is an allergic response in a subject sensitized to αGal.
14. The polymeric glycoconjugate compound for use in a method according to clause 13, wherein said subject has been sensitized to αGal further to a hard tick bite, preferably of the genera *Amblyomma* or *Ixodes,* more preferably selected from *Amblyomma americanum, Ixodes ricinus, Ixodes holocyclus, Amblyomma testudinarium* and members of the *Amblyomma cajennense* complex, such as *Amblyomma sculptum.*
15. The polymeric glycoconjugate compound for use according to any of clauses 13 or 14, wherein said αGal sensitized subject is characterized by an increase of serological levels of anti-aGal IgE antibodies with respect to a non-sensitized subject.
16. The polymeric glycoconjugate compound for use according to any of clauses 13 to 15, wherein said allergic response mediated by anti-aGal immunoglobulins of the IgE isotype occurs further to exposure to a protein with a glycosylation pattern containing the αGal epitope by the oral or parenteral route.
17. The polymeric glycoconjugate compound for use according to any of clauses 7 to 16, wherein further to administration of the compound to the subject said compound removes at least 80%, preferably at least 90% of the serum anti-aGal immunoglobulins of the IgE isotype.
18. The polymeric glycoconjugate compound for use according to any of clauses 7 to 17, wherein said subject is a human subject.
19. The polymeric glycoconjugate compound for use according to any of clauses 7 to 18, wherein said compound is formulated for parenteral administration, preferably for intravenous, intramuscular or subcutaneous administration, more preferably for subcutaneous administration.

The following examples serve to illustrate the present invention and should not be construed as limiting the scope thereof.

### EXAMPLES

### Example 1.- Materials and methods

### Polymeric αGal-glycoconjugates

αGal-glycoconjugates were chemically synthetized from a customized linear Poly-l-lysine and the Galili-epitope (Galα1-3Galβ1-4GlcNAc-) as starting materials as previously described (WO 98/47915, for example the conjugate Type 1 C1g was synthesized from Poly-l-lysine B1c (Example B1) and Galili-epitope A2 (Example A9)). Glycopolymers were composed by a linear polymeric backbone of 1000 ± 10% l-lysine units with 9 (RA0109), 12 (RA0112), 18 (RA0118), and 27 (RA0127) percentage of the lateral lysine chains substituted with the αGal-epitope. Figure 1 provides the general structure of the tested polymeric αGal-glycoconjugates, wherein a: 1000 ± 10% l-lysine units; and r: 0,09 or 0,12 or 0,18 or 0,27 (substituted fraction of αGal epitope). γ-Thiobutyrolactone was used as a linker between the lateral lysine chain and the sugar residue. The synthesis of the final compounds was performed with the same batch of both Poly-l-lysine and the αGal epitope.

The percentage of sugar load (Galili-epitope) in the final polymeric glycoconjugates was determined by quantitative ¹H-NMR using 600 MHz Bruker Avance spectrometer. A direct relation between protons characteristic of Galili-epitope and thioglycerol was established. Proton (5.2 ppm) corresponds to Galili-epitope and proton (2.8 ppm) to thioglycerol. The integration of these signals allowed defining the load relation between thioglycerol and Galili-epitope in the polymers. Resulting αGal-glycoconjugates presented twenty-seven (RA0127, Fig. 12A), eighteen (RA0118, Fig. 12B), twelve (RA0112, Fig. 12C) and nine percentage (RA0109, Fig. 12D) of Galili-epitope load in the final structure.

### Human serum from patients with α-Gal allergy

Human serum was collected from eight αGal-sensitized subjects (PT), which show elevated serological levels of IgE anti-αGal antibodies compared to controls. Serum from eight healthy volunteer subjects were used as controls (CN).

Treatment of human serum with polymeric glycoconjugates

To test the inhibition of anti-αGal antibodies, the αGal-glycoconjugates (RA0109, RA0112, RA0118 and RA0127) were individually incubated (mild agitation) at growing concentrations (5-10-20-50-100 µg/mL) with human serum during 12 h, at 4°C. Phosphate buffered saline (PBS) was used as a vehicle and negative control. After incubation, the mix was centrifuged 10000 g 10 min and the supernatant was used for antibodies determination (n=8).

### α1,3-galactosyltransferase knocked out mice

This study was performed in mice (n = 48, 24 male and 24 female) of 38-44 weeks old, in which the gene coding for the α1,3-galactosyltransferase enzyme had been knocked out (GalT-KO mice), and were derived from a highly inbred colony with a hybrid genetic background (B6xCBAxl29sv) (Costa C *et al.,* 2003). Mice were maintained at specific pathogen-free (SPF) facilities, under controlled temperature (21 ± 1°C), humidity (55 ± 5%) and cycles of light/dark (12/12 h). Food and water were given ad libitum. Teklad global 14% protein (Envigo, Huntingdon, UK) was used as a standard rodent maintenance diet.

### Amblyomma sculptum saliva extract

Saliva was collected from 200 females of unfed *Amblyomma sculptum.* Saliva was lyophilized to yield an amount of 3.11 mg and kept at -20°C until use. Sterile PBS was conveniently used as vehicle to prepare the final aqueous solution injected as allergen to the GalT-KO mice (Araujo RN *et al.,* 2016).

### GalT-KO mice sensitization for IgE anti-αGal production

Induction of IgE anti-aGal antibodies in GalT-KO mice was achieved by immunization with saliva extract from *Amblyomma sculptum* (the extract). GalT-KO mice were randomly separated in three different groups (n=16, Figure 2).

The Group 1 was a double negative control. The Group 2 and 3 were intradermally (id.) immunized with two doses of 20 µg of the extract (days 0 and 7) for mice sensitization. Animals were then immunized with three consecutive doses of 5µg of the extract on days 14, 15 and 16 to induce the production of IgE anti-aGal antibodies. The Group 2 was a negative control for the treatment, the animals were only treated with the vehicle (PBS sc.). In the Group 3, animals were treated with three consecutives subcutaneous RA0118 doses (10 mg/Kg) on days 16 (4 h after last challenge), 17 and 18. The induction procedure with saliva extract from *Amblyomma sculptum* was repeated in two animals of Group 3 two weeks after last treatment with RA0118 (day 26). Animal blood was collected by controlled-submandibular bleeding (Golde WT *et al.,* 2005) on days -3 (baseline), 16 (4 h after challenge), 18 (4h after treatment) and 28 (after rechallenge, two animals Group 3). Fresh blood from 18 animals (six from each experimental group, n=6) was used to white blood cell (WBC) determination by Flow Cytometry. The sera from the remaining 30 animals was stored at -20°C and divided for antibodies and cytokine determination accordingly.

### IgE, IgG, IgM and IgA anti-αGal antibodies by ELISA

Anti-aGal antibodies were determined following the general protocol previously described (Bello-Gil D *et al.,* 2019). Briefly, Nunc MaxiSorp^{™} 96-well flat bottom plates (Thermo Fisher Scientific, Waltham, MA, USA) were coated with 2.5 µg/mL of Galα1-3Galβ1-4GlcNAc glycan conjugated to human serum albumin (Dextra Laboratories Ltd, Reading, UK). After coating, the plates were washed three times with PBS with 0.5% (v/v) Tween-20, and then blocked for 1 h at 4°C with 0.05% (v/v) Tween-20 in PBS. Washing steps were repeated, and serum samples diluted in PBS (1:100 for IgM and IgG, 1:25 for IgA and 1:10 for IgE) were added to the wells and incubated for 1 h at 25°C. After washing, the plates were incubated for 1 h at 25°C with the corresponding horseradish peroxidase (HRP)-labeled secondary antibodies diluted in PBS (Anti-Mouse IgG- HRP Invitrogen 62-6520; Anti-Mouse IgM- HRP Invitrogen 62-6820; Anti-Mouse IgE- HRP Invitrogen PA1-84764; Anti-Human IgG- HRP Invitrogen A18811; Anti-Human IgM- HRP Invitrogen A18841; Anti-Human IgA- HRP Invitrogen PA1-74395; Anti-Human IgE- HRP Invitrogen A18793 (Thermo Fisher Scientific, Waltham, Massachusetts, US)). After another round of washing, an HRP substrate was added to the wells. The reaction was stopped with 3N HCl, and the resulting absorbance was registered at 492 nm using a microplate reader (BioTek, Winooski, VT, USA).

### Total circulating mouse IgM, IgG1, IgG2a, IgG2b, IgG3 and IgE by ELISA

Total serum immunoglobulins (IgM, IgE, IgG1, IgG2a, IgG2b and IgG3) were determined using the commercial RayBio^{®} mouse ELISA kit following manufacturer instructions (RayBiotech, GA, USA).

### Cytokine profile

Inflammatory cytokines (GCSF, GM-CSF, IFN-γ, IL-1α (IL-1 F1), IL-1 beta (IL-1 F2), IL-10, IL-12 p70, IL-13, IL-15, IL-17A, IL-2, IL-21, IL-23 p19, IL-3, IL-4, IL-5, IL-6, IL-7, IL-9, TNF α) were assessed according to the Quantibody^{®} Mouse Interleukin Array kit following manufacturer instructions (RayBiotech, GA, USA).

### White blood cells by Flow Cytometry

Flow cytometry analysis was performed on a Gallios analyser (Beckman Coulter, IN, USA) equipped with a violet (405nm), a blue (488 nm) and a red (633 nm) solid-state lasers as an exciting source. Purified Rat Anti-Mouse CD16/CD3 antibody was used to block non-antigen-specific binding (#553142, Beckton Dickinson, CA, USA). Immunophenotyping of different WBC populations, for every experimental group (n=6), was performed using a panel of fluorochrome conjugated antibodies, all of them purchased from Beckton Dickinson (CA, USA), see Table 1 below. Fluorescence was collected through the next band pass filters for each indicated surface cell marker. Data were analyzed using KALUZA software (Beckman Coulter).

**Table 1. - Fluorochrome conjugated antibodies used for immunophenotyping of different WBC populations.**

| Detector | Band Pass Filter | Fluorochrome | Antibody | Reference |
|---|---|---|---|---|
| FL1 | 525/40 | FITC | Rat Anti-Mouse CD45 | 561088 |
| FL2 | 575/30 | PE | Rat Anti-CD 11b | 561689 |
| FL3 | 620/30 | PE-CF594 | Rat Anti-Mouse CD8a | 562315 |
| FL4 | 695/40 | BB700 | Rat Anti-Mouse CD193 | 742079 |
| FL5 | 755LP | PE-Cy7 | Rat Anti-Mouse CD19 | 561739 |
| FL6 | 660/20 | APC | Rat Anti-Mouse Ly-6G | 560599 |
| FL7 | 725/20 | APC-R700 | Rat Anti-Mouse CD5 | 565505 |
| FL8 | 755LP | PC-H7 | Rat Anti-Mouse CD4 | 560246 |
| FL9 | 450/50 | BV421 | Rat Anti-Mouse IgE | 564207 |
| FL10 | 550/40 | V500 | Syrian Hamster anti-Mouse CD3e | 560771 |

### Statistics

*In vitro* studies of anti-aGal antibodies removal are the result of three independent experiments using αGal-sensitized and control subjects (n=8-10). In the ELISA of anti-aGal, serum samples were determined by triplicate and values (optical density units) were either normalized with respect to the maximum signal obtained in each experimental run or expressed as median of removal (%), referred to the same serum treated with the vehicle (PBS) under identical conditions.

In the *in vivo* model, animals were randomly ubicated (sex parity) in the three experimental groups (n=16). Total immunoglobulins in mouse samples were measured by ELISA (n=7). Serological levels of cytokines were followed by protein array (n=6). The standards of cytokines were printed in 4 replicates and the binding results in relative fluorescence units for every experimental day (-3, 16 and 18) were expressed as fold-change between days. In every single FACS determination of WBC were recorded about 150.000 total events, of which 50.000 were CD45 positive.

GraphPad Prism statics software (GraphPad Software Inc., San Diego, CA, USA) was used for analysis and data graphing. The Gaussian distribution of data was checked by the D'Agostino-Pearson omnibus normality test (alpha = 0.05), and homogeneity of variances was determined by the F test (alpha = 0.05). Most of the statistical analyses were performed using paired parametric t-tests. The Wilcoxon matched-pairs signed rank and Mann-Whitney tests (unpaired data analysis) were used as non-parametric tests when data did not follow a Gaussian distribution. Tukey and Sidak were used as multiple comparison tests. Grubbs' test (extreme studentized deviate) was used to determine significant outliers in the data (alpha = 0.05). Differences were considered statistically significant when p < 0.05 (*: p < 0.05; **: p < 0.01; ***: p < 0.001; ****: p < 0.0001).

### Example 2.- In vitro inhibition of IgE anti-aGal antibodies

To evaluate the inhibition and removal of IgE anti-aGal antibodies, the different polymeric αGal-glycoconjugates (RA0109, RA0112, RA0118, RA0127) were combined, at growing concentration (0.005-0.01-0.02-0.05-0.1 mg/mL), with human serum of ten αGal-sensitized (PT). Patients showed significant elevated circulating levels of IgE anti-aGal antibodies compared to controls (CN) (p < 0.0001), as shown in Figure 3. PT also showed higher significant serological levels (p < 0.001) for the rest of the anti-aGal isotypes (IgM, IgG and IgA) compared to CN (Figure 3). All the statistical analysis referred to the inhibition assays are summarized in Table 2.

**Table 2. - Summary of statistical analysis**

| | | **Serum from αGal-sensitized subjects (PT)** | | | | | |
|---|---|---|---|---|---|---|---|
| | | **RA0127 vs RA0118** | **RA0127 vs RA0112** | **RA0118 vs RA0112** | **RA0127 vs RA0109** | **RA0118 vs RA0109** | **RA0112 vs RA0109** |
| **IgE** | **0.005** | ns | ns | ns | **** | **** | **** |
| | **0.01** | ns | ns | ns | **** | **** | **** |
| | **0.02** | ns | ns | ns | **** | **** | **** |
| | **0.05** | ns | ns | ns | **** | **** | **** |
| | **0.1** | ns | ns | ns | **** | **** | **** |
| **IgG** | **0.005** | ns | ns | ns | **** | **** | **** |
| | **0.01** | ns | ns | ns | **** | **** | **** |
| | **0.02** | ns | ns | ns | **** | **** | **** |
| | **0.05** | ns | ns | ns | **** | **** | **** |
| | **0.1** | ns | ns | ns | **** | **** | **** |
| **IgM** | **0.005** | ns | ns | ns | **** | **** | **** |
| | **0.01** | ns | ns | ns | **** | **** | **** |
| | **0.02** | ns | ns | ns | **** | **** | **** |
| | **0.05** | ns | ns | ns | **** | **** | **** |
| | **0.1** | ns | ns | ns | **** | **** | **** |
| **IgA** | **0.005** | ns | ns | ns | **** | **** | **** |
| | **0.01** | ns | ns | ns | **** | **** | **** |
| | **0.02** | ns | ns | ns | **** | **** | **** |
| | **0.05** | ns | ns | ns | **** | **** | **** |
| | **0.1** | ns | ns | ns | **** | **** | **** |

| | | | | | | | |
|---|---|---|---|---|---|---|---|
| n.s.(non-significant); **** (p < 0.0001) | | | | | | | |

Overall, the exposure to the polymeric glycoconjugates mostly inhibited anti-aGal IgE and IgM isotypes, with a lower inhibition effect on the IgA and IgG isotypes respectively (Figure 4). This general trend was very similar for all the glycoconjugates at the different assessed concentrations (Figure 4). For all the isotypes there was a direct relation between polymer concentration and the percentage of isotype inhibition. This effect was clearer for RA0109, where an increase in polymer concentration resulted in a proportional increase on the isotype removal (Figure 4).

The glycoconjugate RA0109 showed significantly lower capacity to inhibit the anti-aGal antibodies compared to RA0112, RA0118 and RA0127 (Figure 4-5, Table 2). Surprisingly, the inventors found that the effect on isotypes inhibition was rarely similar (ns) for RA0112, RA0118 and RA0127, despite of the differences on the percentage of the αGal-epitope substitution in the final polymer (Figure 4-5, Table 2). Indeed, it was surprisingly found that RA0112 and RA0118 (12 and 18% of αGal-epitope substitution, respectively) showed the best inhibition results for most of the anti-aGal isotypes and concentrations evaluated, at the same level of RA0127 (Figure 5). This finding was observed even at the lower concentration assessed.

Regarding the inhibition of IgE isotype, RA0109 at 0.1 mg/mL inhibited about 50% of circulating anti-aGal IgE, whereas RA0112, RA0118 and RA0127 inhibited more than 90% of circulating anti-aGal IgE even at the lowest polymer concentration evaluated (Figure 5). Hence, the sugar load in the RA0109 at the assessed polymer concentrations was not effective to achieve a complete inhibition of the IgE isotype in PT. Surprising, adding only a three percentage of αGal-load to the RA0109 structure (the RA0112 glycoconjugate) was enough to achieve almost a complete IgE inhibition.

In general terms, inhibition of IgM followed a similar trend compared to the IgE isotype for RA0112, RA0118 and RA0127 glycoconjugates (Figure 4-5, Table 2). For anti-aGal IgA, the trend for these glycopolymers was also very similar (ns), reaching a 95% of IgA removal at 0.1 mg/ml. Finaly, for IgG, although RA0112, RA0118 and RA0127 followed a similar trend (ns), the authors found significant lower percentages of removal for most of the assessed polymer concentrations compared to the rest of isotypes.

It was completely unexpected that anti-aGal IgE outcompetes IgG and IgA, showing a similar behavior to IgM isotype. IgE is in a monomeric presentation, like IgG and IgA. IgE antibodies are evolutionarily conserved and heavily glycosylated heterotetramers with the ε-heavy chain containing four constant domains. Structurally, IgE differs in important ways from IgG (Sutton BJ *et al.,* 2019) but also shares the highest homology with IgG compared to the rest of isotypes. The Cε3 and Cε4 domains have a sequence homology of 32% and similar quaternary structure as the Cγ2 and Cγ3 domains of IgG. As a substitute for the flexible hinge region of IgG, the IgE has an additional Cε2 domain located at the same position (Wurzburg BA *et al.*, 2000). Thus, a similar trend to IgG was expected for IgE isotype.

Although the anti-αGal antibodies are naturally present in all healthy humans (Galili U, 2015), αGal sensitized individuals have additional anti-αGal antibodies that contribute to this pool. As shown in this work the contribution of induced anti-αGal IgG to the natural anti-αGal IgG pool is significant. We also demonstrated that induced anti-αGal IgG antibodies in our *in vivo* model are of a different subclass than the natural ones (see Example 3 below).

### Example 3.- In vivo inhibition of anti-aGal IgE antibodies

### Results

GalT-KO mice were sensitized to αGal glycan residues by intradermal immunization of saliva extract from *Amblyomma sculptum* to induce augmented serological levels of IgE anti-αGal antibodies. The objective was to explore a therapeutic solution for removing *in vivo* these antibodies with the polymeric αGal-glycoconjugate RA0118, which demonstrated the best inhibition capacity among the assessed glycopolymers in the *in vitro* studies.

A model of mice sensitization to αGal was developed. It was achieved a clear induction of the IgE anti-αGal antibodies in all the animals of Group 2 and 3 (Figure 2 and 6). This induction was also accompanied with augmented levels of IgG and IgM anti-αGal antibodies. The induction procedure with saliva extract from *Amblyomma sculptum* was repeated in two animals of Group 3 two weeks after last treatment with RA0118 (day 26). It was induced again elevated levels of IgE anti-αGal antibodies (day 28, Figure 6). We achieve to mimic the mechanism of IgE anti-αGal antibodies production reported in humans after tick bites, demonstrating the validity of the model.
Additionally, we also evaluated the impact of GalT-KO mice sensitization in the total serological levels of immunoglobulins and proinflammatory cytokines. Regarding circulating immunoglobulins, it was observed a slight but significant increase in the serological levels of total IgE and IgG2b isotype for Group 2 and 3 after mice sensitization (Figure 7). Importantly, these trends in both experimental groups were clearly associated with the induction on day 16 of augmented levels of anti-αGal antibodies for the IgE and IgG isotype, respectively. The treatment with RA0118 (day 18) was associated with a clear decrease in Group 3 of the total levels of anti-αGal IgE and IgG antibodies respect to controls (treatment with vehicle in Group 2). Treatment with RA0118 achieved an *in vivo* removal of more than 70% of the induced anti-αGal IgE antibodies in the serum of aGal sensitized GalT-KO mice, (Figure 8).

Interestingly, the treatment in Group 3 with RA0118 also decreased the total serological levels of IgG2b, restoring the baseline concentration for this isotype. Therefore, the augmented serological levels of total IgG2b isotype on day 16 is a result of anti-αGal IgG contribution after the protocol of mice sensitization.

Most of the assessed isotypes (total IgM, IgG1, IgG2a, IgG3) did not show significant difference among the evaluated days for all the experimental groups (Figure 9). Only total IgM showed a decrease in Group 3 (Figure 9) from day 16 to 18 due to the treatment. As shown in the *in vitro* studies (Figure 4-5), RA0118 can inhibit anti-αGal IgM antibodies. These antibodies are highly represented in the natural repertoire of circulating IgM in GalT-KO mice (Bello-Gil D *et al.,* 2019); thus, its removal can significantly impact the total serological levels of the IgM isotype.

Regarding cytokine profile, it was observed the same trend for most of the assessed cytokines, with no differences between days and experimental groups. The only cytokine showing changes between experimental groups was IL-1β. On day 16, the serological levels of IL-1β were increased 45- and 59-fold respect to baseline in the Group 2 and Group 3, respectively, as a result of mice sensitization. All mice from control (Group 1) showed very low serological levels of this cytokine without significant variation during the procedure (Figure 10).

Finally, immunophenotyped WBC populations were assessed by FACS. During αGal sensitization and anti-αGal IgE induction in GalT-KO mice, the major change in WBC population was registered for basophils. We detected a significantly higher fluorescence intensity on day 16 for Group 2 and Group 3 compared to control (Group 1) as a result of a putative overexpression of IgE isotype in the membrane of basophils. On day 18, the augmented levels start to decrease without differences between Group 2 and 3. The rest of evaluated WBC populations remained without differences among days and experimental groups.

### Discussion

The *in vitro* proof of concept was validated *in vivo.* We developed a model to αGal sensitization in GalT-KO mice, achieving a clear induction of the IgE anti-aGal antibodies, together with IgG and IgM. RA0118 showed a high efficacy as therapy for intracorporeally removing IgE anti-αGal antibodies. These results also confirmed the implication of saliva components from tick *Amblyomma sculptum* in the mechanism of anti-αGal induction, in accordance with previous research works (Araujo RN *et al.,* 2016; Commins SP, Karim S, 2017). Induction of antibodies was accompanied with significant elevated levels of IL-1β in the Group 2 and Group 3 compared to control. This potent proinflammatory cytokine has been described in many allergic disorders (Han MW *et al.,* 2019). However, compounds present in the tick saliva has been described to reduce the production of pro-inflammatory cytokines such as IL-1β (Kotál J *et al.,* 2005). This finding was not observed in our animal model.

Additionally, we demonstrated an activation of basophils in Group 2 and Group 3. It was observed a higher expression of IgE on basophils in these experimental groups compared to the control group. In patients with delayed anaphylaxis to red meat, basophils activation appears to be a predictor of clinical reaction severity (Kotál J *et al.,* 2005). Therefore, we were able to induce a specific activation of basophils in a similar manner to that observed in patients with α-Gal syndrome. Overall, the objective to mimic human condition by inducing high serological levels of anti-αGal IgE antibodies and basophils activation was clearly achieved.

The immune stimulation with the saliva extract of *Amblyomma sculptum* was not restricted to the production of IgE, but also to the anti-αGal IgG and IgM isotype. Elevated titers of anti-αGal IgG antibodies have been previously described in IgE-positive subjects (Rispens T *et al.,* 2013). Specifically, the authors found proportionally more IgG1 anti-αGal in IgE-positive subjects against a background of IgG2 production specific for αGal. These results suggest that IgE to a carbohydrate antigen can be formed (probably as part of a glycoprotein or glycolipid) even against a background of bacterial immune stimulation with essentially the same antigen (Rispens T *et al.*, 2013).

As described in Example 2, human subjects sensitized to αGal showed augmented levels of anti-αGal antibodies compared to controls for all the assessed isotypes (IgE, IgG, IgM and IgA). Interesting, the *in vivo* results indicate that the IgE anti-αGal response induced by tick saliva is characterized by augmented levels of IgG anti-αGal too. Typically, allergens are glycoproteins. Thus, they can induce the formation of IgG as well as IgE antibodies. This relationship between IgE and IgG antibodies in type I allergy has been previously described (Vailes LD *et al.,* 2001). Also, the augmented levels of IgE and IgG anti-αGal were highly correlated to the increase of total serological levels of IgE and IgG2b isotype, respectively. The significant input of IgE anti-αGal to the total serological level of IgE was expected taking in consideration the relatively low serological concentration of this isotype in healthy animals. However, the significant input of anti-αGal IgG to the total serological levels of IgG2b isotype was surprising, indicating a preferential induction of this IgG-subclass after animal sensitization. GalT-KO mice spontaneously produce elevated levels of natural anti-αGal IgG and IgM antibodies (Bello-Gil D *et al.,* 2019). However, most of these antibodies are IgG3-subclass (unpublished data). Therefore, induced and natural IgG anti-αGal are mostly represented by a different IgG-subclass. This difference may be the result of different mechanism for antigen presentation. Consequently, two types of immune response to αGal epitopes can be distinguished in the animal model: a 'typical' IgG3 response, presumably in response to the continues stimulation by the gut microbiota (natural antibodies), and an 'atypical', Th2-like response after mice sensitization, leading to IgG2b and IgE (induced antibodies) in addition to the "natural" IgG3. A similar process has been described in human subjects sensitized to αGal residues after tick bites (Rispens T *et al.*, 2013).

### REFERENCES

Deal CE, Balazs AB (2015) Engineering humoral immunity as prophylaxis or therapy. Curr Opin Immunol. 35:113-22, doi: 10.1016/j.coi.2015.06.014.
Wang L, Wang FS, Gershwin ME (2015) Human autoimmune diseases: a comprehensive update. J Intern Med. 278(4):369-95, doi: 10.1111/joim.12395.
Kubo M (2017) Innate and adaptive type 2 immunity in lung allergic inflammation. Immunol Rev. 278(1):162-172, doi: 10.1111/imr.12557.
Giannoccaro MP, Crisp SJ, Vincent A (2018) Antibody-mediated central nervous system diseases. Brain and Neuroscience Advances 2: 1-10, doi: 10.1177/2398212818817497.
Paschou SA, Papadopoulou-Marketou N, Chrousos GP, Kanaka-Gantenbein C (2018) On type 1 diabetes mellitus pathogenesis. Endocr Connect. 7(1): R38-R46, doi: 10.1530/EC-17-0347.
Scott DL, Wolfe F, Huizinga TW (2010) Rheumatoid arthritis. Lancet. 376(9746):1094-108, doi: 10.1016/S0140-6736(10)60826-4.
Rivas-Larrauri F, Yamazaki-Nakashimada MA (2016) Systemic lupus erythematosus: Is it one disease? Reumatol Clin. 12(5):274-81, doi: 10.1016/j.reuma.2016.01.005.
Liu CY, Zhu J, Zheng XY, Ma C, Wang X (2017) Anti-N-Methyl-D-aspartate Receptor Encephalitis: A Severe, Potentially Reversible Autoimmune Encephalitis. Mediators Inflamm. 2017:6361479, doi: 10.1155/2017/6361479.
Hu J, Chen J, Ye L, Cai Z, Sun J, Ji K (2018) Anti-IgE therapy for IgE-mediated allergic diseases: from neutralizing IgE antibodies to eliminating IgE+ B cells. Clin Transl Allergy. 8:27, doi: 10.1186/s13601-018-0213-z.
European Academy for Allergy and Clinical Immunology (2016) Advocacy Manifesto: Tackling the Allergy Crisis in Europe - Concerted Policy Action Needed. http://www.eaaci.org/outreach/public-declarations/3243-advocacy-manifesto-tackling-the-allergy-crisis-in-europe.
Ring J, Akdis C, Lauener R, Schäppi G, Traidl-Hoffmann C, Akdis M, et al. (2014) Global Allergy Forum and Second Davos Declaration 2013 Allergy: Barriers to cure--challenges and actions to be taken. Allergy 69(8):978-82, doi: 10.1111/all.12406.
Kopp MV (2011) Omalizumab: Anti-IgE therapy in allergy. Curr Allergy Asthma Rep. 11(2):101-6, doi: 10.1007/s11882-010-0173-4.
Galili U (2013) Anti-Gal: an abundant human natural antibody of multiple pathogeneses and clinical benefits. Immunology. 140(1):1-11, doi: 10.1111/imm.12110.
Galili U (2015) Significance of the evolutionary α1,3-galactosyltransferase (GGTA1) gene inactivation in preventing extinction of apes and old world monkeys. J Mol Evol. 80(1):1-9, doi: 10.1007/s00239-014-9652-x.
Bello-Gil D, Audebert C, Olivera-Ardid S, Perez-Cruz M, Even G, Khasbiullina N et al. (2019) The Formation of Glycan-Specific Natural Antibodies Repertoire in GalT-KO Mice Is Determined by Gut Microbiota. Front Immunol. 10:342, doi: 10.3389/fimmu.2019.00342.
Sandrin MS, Vaughan HA, Dabkowski PL, McKenzie IF (1993) Anti-pig IgM antibodies in human serum react predominantly with Gal(alpha 1-3)Gal epitopes. Proc Natl Acad Sci USA 90:11391-5, doi: 10.1073/pnas.90.23.11391.
Parker W, Lin SS, Yu PB, Sood A, Nakamura YC, Song A, et al. (1999) Naturally occurring anti-alpha-galactosyl antibodies: relationship to xenoreactive anti-alpha-galactosyl antibodies. Glycobiology 9:865-73, doi: 10.1093/glycob/9.9.865.
Commins SP, Platts-Mills TAE (2013) Delayed anaphylaxis to red meat in patients with IgE specific for galactose alpha-1,3-galactose (alpha-gal). Curr Allergy Asthma Rep. 13(1):72-7, doi: 10.1007/s11882-012-0315-y.
Davies JM, Platts-Mills TAE, Aalberse RC (2013) The enigma of IgE+ B-cell memory in human subjects. J Allergy Clin Immunol. 131(4):972-6, doi: 10.1016/j.jaci.2012.12.1569.
Berg EA, Platts-Mills TAE, Commins SP (2014) Drug allergens and food--the cetuximab and galactose-α-1,3-galactose story. Ann Allergy Asthma Immunol. 112(2):97-101, doi: 10.1016/j.anai.2013.11.014.
Commins SP, Satinover SM, Hosen J, Mozena J, Borish L, Lewis BD, et al. (2009) Delayed anaphylaxis, angioedema, or urticaria after consumption of red meat in patients with IgE antibodies specific for galactose-alpha-1,3-galactose. J Allergy Clin Immunol. 123(2):426-33, doi: 10.1016/j.jaci.2008.10.052.
Platts-Mills TAE, Schuyler AJ, Hoyt AE, Commins SP (2015) Delayed Anaphylaxis Involving IgE to Galactose-alpha-1,3-galactose. Curr Allergy Asthma Rep. 15(4):12, doi: 10.1007/s11882-015-0512-6.
Commins SP, Jerath MR, Cox K, Erickson LD, Platts-Mills TAE (2016) Delayed anaphylaxis to alpha-gal, an oligosaccharide in mammalian meat. Allergol Int. 65(1): 16-20, doi: 10.1016/j.alit.2015.10.001.
Costa C, Brokaw JL, Wang Y, Fodor WL (2003) Delayed rejection of porcine cartilage is averted by transgenic expression of alpha1,2-fucosyltransferase. FASEB J., doi: 10.1096/fj.02-0630fje.
Golde WT, Gollobin P, Rodriguez LL (2005) A rapid, simple, and humane method for submandibular bleeding of mice using a lancet. Lab Anim. 34:39-43, doi: 10.1038/laban1005-39.
Katopodis AG, Warner RG, Duthaler RO, Streiff MB, Bruelisauer A, Kretz O, et al. (2002) Removal of anti-Galalpha1,3Gal xenoantibodies with an injectable polymer. J Clin Invest. 110(12):1869-77, doi: 10.1172/JCI16526.
Muthana SM, Xia L, Campbell CT, Zhang Y, Gildersleeve JC (2015) Competition between serum IgG, IgM, and IgA anti-glycan antibodies. PLoS One. 10(3):e0119298, doi: 10.1371/journal.pone.0119298.
Eisen HN (2014) Affinity enhancement of antibodies: how low-affinity antibodies produced early in immune responses are followed by high-affinity antibodies later and in memory B-cell responses. Cancer Immunol Res. 2(5):381-92, doi: 10.1158/2326-6066.CIR-14-0029.
Sutton BJ, Davies AM, Bax HJ, Karagiannis SN (2019) IgE Antibodies: From Structure to Function and Clinical Translation. Antibodies 8(1) 19, doi: 10.3390/antib8010019
Wurzburg BA, Garman SC, Jardetzky TS (2000) Structure of the human IgE-Fc C epsilon 3-C epsilon 4 reveals conformational flexibility in the antibody effector domains. Immunity 13(3):375-85, doi: 10.1016/S1074-7613(00)00037-6.
Rispens T, Derksen NI, Commins SP, Platts-Mills TAE, Aalberse RC (2013) IgE production to α-gal is accompanied by elevated levels of specific IgG1 antibodies and low amounts of IgE to blood group B. PLoS One 8(2):e55566, doi: 10.1371/journal.pone.0055566.
Vailes LD, Perzanowski MS, Wheatley LM, Platts-Mills TAE, Chapman MD (2001) IgE and IgG antibody responses to recombinant Alt a 1 as a marker of sensitization to Alternaria in asthma and atopic dermatitis. Clin Exp Allergy. 31(12):1891-5, doi: 10.1046/j.1365-2222.2001.00745.x.
Commins SP, Karim S (2017) Development of a novel murine model of alpha-gal meat allergy. JACI, volume 139, Issue 2, Supplement, Page AB193, doi: 10.1016/j.jaci.2016.12.628.
Araujo RN, Franco PF, Rodrigues H, Santos LCB, McKay CS, Sanhueza CA, et al. (2016) Amblyomma sculptum tick saliva: α-Gal identification, antibody response and possible association with red meat allergy in Brazil. Int J Parasitol. 46(3):213-220, doi: 10.1016/j.ijpara.2015.12.005.
Krause K, Metz M, Makris M, Zuberbier T, Maurer M (2012) The role of interleukin-1 in allergy-related disorders. Curr Opin Allergy Clin Immunol. 12(5):477-84, doi: 10.1097/ACI.0b013e3283574d0c.
Han MW, Kim SH, Oh I, Kim YH, Lee J (2019) Serum IL-1β can be a biomarker in children with severe persistent allergic rhinitis. Allergy Asthma Clin Immunol. 15:58, doi:10.1186/s13223-019-0368-8.
Kotál J, Langhansová H, Lieskovská J, Andersen JF, Francischetti IM, Chavakis T, et al. (2015) Modulation of host immunity by tick saliva. J Proteomics. 128:58-68. doi: 10.1016/j.jprot.2015.07.005
Choudhary SK, Commins SP (2017) Association of Alpha-gal Red Meat Allergy Severity with Concentration of Antigen in Basophil Activation. JACI, volume 139, Issue 2, Supplement, Page AB125, doi: 10.1016/j.jaci.2016.12.406.
Commins SP, James HR, Stevens W, Pochan SL, Land MH, King C, et al. (2014) Delayed clinical and ex vivo response to mammalian meat in patients with IgE to galactose-alpha-1,3-galactose. J Allergy Clin Immunol. 134(1):108-15. doi: 10.1016/j.jaci.2014.01.024.
Fischer J, Eberlein B, Hilger C, et al. Alpha-gal is a possible target of IgE-mediated reactivity to antivenom. Allergy. 2017;72(5):764-771. doi:10.1111/all.13073.
Wilson JM, Schuyler AJ, Schroeder N, Platts-Mills TAE. Galactose-α-1,3-Galactose: Atypical Food Allergen or Model IgE Hypersensitivity?. Curr Allergy Asthma Rep. 2017; 17(1):8. doi:10.1007/s11882-017-0672-7.
Gronlund H, Adédoyin J, Commins SP, Platts-Mills TAE, van Hage M. The carbohydrate galactose-alpha-1,3-galactose is a major IgE-binding epitope on cat IgA. J Allergy Clin Immunol. 2009;123(5):1189-1191. doi:10.1016/j.jaci.2009.03.011
Mukai, K., Tsai, M., Starkl, P. et al. IgE and mast cells in host defense against parasites and venoms. Semin Immunopathol 38, 581-603 (2016). https://doi.org/10.1007/s00281-016-0565-1.
Fitzsimmons CM, Falcone FH, Dunne DW. Helminth Allergens, Parasite-Specific IgE, and Its Protective Role in Human Immunity. Front Immunol. 5:61. (2014) doi:10.3389/fimmu.2014.00061
Kwak M, Somerville C, van Nunen S (2018). A novel Australian tick Ixodes (Endopalpiger) australiensis inducing mammalian meat allergy after tick bite. Asia Pacific Allergy. 8 (3): e31. doi:10.5415/apallergy.2018.8.e31.
Raghavan, R. K., A. T. Peterson, M. E. Cobos, R. Ganta, and D. Foley. 2019. Current and Future Distribution of the Lone Star Tick, Amblyomma americanum (L.) (Acari: Ixodidae) in North America. PLOS ONE. 14: e0209082.
Wilson JM, Platts-Mills TAE. Red meat allergy in children and adults. Curr Opin Allergy Clin Immunol. 2019;19(3):229-235. doi:10.1097/ACI.0000000000000523
Commins SP, Platts-Mills TAE. Tick bites and red meat allergy. Curr Opin Allergy Clin Immunol. 2013;13(4):354-359. doi:10.1097/ACI.0b013e3283624560.
Gonzalez-Quintela A, Dam Laursen AS, Vidal C, Skaaby T, Gude F, Linneberg A. IgE antibodies to alpha-gal in the general adult population: relationship with tick bites, atopy, and cat ownership. Clin Exp Allergy. 2014;44(8):1061-1068. doi:10.1111/cea.12326
Sagurova I, Ludwig A, Ogden NH, Pelcat Y, Dueymes G, Gachon P. Predicted Northward Expansion of the Geographic Range of the Tick Vector Amblyomma americanum in North America under Future Climate Conditions. Environ Health Perspect. 2019 Oct;127(10):107014. doi: 10.1289/EHP5668
Sonenshine DE. Range Expansion of Tick Disease Vectors in North America: Implications for Spread of Tick-Borne Disease.Int J Environ Res Public Health. 2018 Mar 9;15(3):478. doi: 10.3390/ijerph15030478.
Molaei G, Little EAH, Williams SC, Stafford KC Bracing for the Worst - Range Expansion of the Lone Star Tick in the Northeastern United States. N Engl J Med. 2019 Dec 5;381(23):2189-2192. doi: 10.1056/NEJMp1911661.
Hilger, C., Fischer, J., Wolbing, F. et al. Role and Mechanism of Galactose-Alpha-1,3-Galactose in the Elicitation of Delayed Anaphylactic Reactions to Red Meat. Curr Allergy Asthma Rep 19, 3 (2019). https://doi.org/10.1007/s11882-019-0835-9.
Tjernberg I, Hamsten C, Apostolovic D, van Hage M (2017) IgE reactivity to α-Gal in relation to Lyme borreliosis. PLoS ONE 2017, 12(9): e0185723. https://doi.org/10.1371/journal.pone.0185723
Hussain R, Dawood G, Abrar N, et al. Selective increases in antibody isotypes and immunoglobulin G subclass responses to secreted antigens in tuberculosis patients and healthy household contacts of the patients. Clin Diagn Lab Immunol. 1995;2(6):726-732.
Overdijk MB, Verploegen S, Ortiz Buijsse A, et al. Crosstalk between human IgG isotypes and murine effector cells. J Immunol. 2012;189(7):3430-3438. doi:10.4049/jimmunol.1200356.
Jefferis J., "Glycosylation as a strategy to improve antibody-based therapeutics", 2009, Nature Reviews Drug Discovery 8: 226-234.
Arnold JN, Wormald MR, Suter DM, et al. Human serum IgM glycosylation: identification of glycoforms that can bind to mannan-binding lectin. J Biol Chem. 2005;280(32):29080-29087. doi:10.1074/jbc.M504528200.
Mattu TS, Pleass RJ, Willis AC, et al. The glycosylation and structure of human serum IgA1, Fab, and Fc regions and the role of N-glycosylation on Fcα receptor interactions. J Biol Chem. 1998;273(4):2260-2272. doi:10.1074/jbc.273.4.2260.
Nettleton MY, Kochan JP. Role of glycosylation sites in the IgE Fc molecule. Int Arch Allergy Immunol. 1995;107(1-3):328-329. doi:10.1159/000237017.
Wilson JM, Platts-Mills TAE. Meat allergy and allergens. Mol Immunol. 2018;100:107-112. doi:10.1016/j.molimm.2018.03.018.
Gabrielli A, Candela M, Pisani E, et al. Antibodies against terminal galactosyl (alpha 1-3) galactose epitopes in systemic sclerosis (scleroderma). Clin Exp Rheumatol. 1992;10(1):31-36.
Davin JC, Malaise M, Foidart J, Mahieu P. Anti-alpha-galactosyl antibodies and immune complexes in children with Henoch-Schonlein purpura or IgA nephropathy. Kidney Int. 1987;31(5):1132-1139. doi:10.1038/ki.1987.119.
D'Alessandro M, Mariani P, Lomanto D, Bachetoni A, Speranza V. Alterations in serum anti-alpha-galactosyl antibodies in patients with Crohn's disease and ulcerative colitis. Clin Immunol. 2002;103(1):63-68. doi:10.1006/clim.2001.5180.
Stanek G, Strle F. Lyme borreliosis-from tick bite to diagnosis and treatment. FEMS Microbiol Rev. 2018;42(3):233-258. doi:10.1093/femsre/fux047.
Kullberg BJ, Vrijmoeth HD, van de Schoor F, Hovius JW. Lyme borreliosis: diagnosis and management. BMJ. 2020;369:m1041. Published 2020 May 26. doi:10.1136/bmj.m1041.
Hadjichristidis N, Iatrou H, Pitsikalis M, Sakellariou G. Synthesis of well-defined polypeptide-based materials via the ring-opening polymerization of alpha-amino acid N-carboxyanhydrides. Chem Rev. 2009;109(11):5528-5578. doi:10.1021/cr900049t.
Duthaler RO, Ernst B, Fischer R, et al. In vivo neutralization of naturally existing antibodies against linear alpha(1,3)-galactosidic carbohydrate epitopes by multivalent antigen presentation: a solution for the first hurdle of pig-to-human xenotransplantation. Chimia (Aarau). 2010;64(1-2):23-28. doi:10.2533/chimia.2010.23.
Homann A, Röckendorf N, Kromminga A, Frey A, Platts-Mills TAE, Jappe U. Glycan and Peptide IgE Epitopes of the TNF-alpha Blockers Infliximab and Adalimumab - Precision Diagnostics by Cross-Reactivity Immune Profiling of Patient Sera. Theranostics. 2017;7(19):4699-4709. Published 2017 Oct 17. doi:10.7150/thno.20654.
Platts-Mills TAE, Li RC, Keshavarz B, Smith AR, Wilson JM. Diagnosis and Management of Patients with the α-Gal Syndrome. J Allergy Clin Immunol Pract. 2020;8(1):15-23.e1. doi:10.1016/j.jaip.2019.09.017.

## Claims

1. A polymeric glycoconjugate compound comprising a terminal alpha-galactosyl moiety,
wherein said compound comprises a poly-L-lysine backbone and is represented by formula (I): 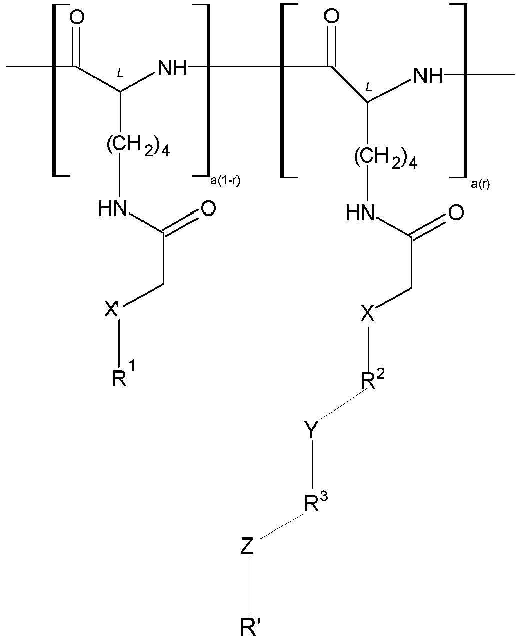
wherein R¹ is - hydrogen, alkyl, alkenyl, alkynyl, alkoxy, acyl, amine, carboxyl, carboxyl ester, cycloalkyl, cycloalkenyl, heterocycloalkyl, heterocycloalkenyl, aryl, and heteroaryl;
X, X', and Y are each independently -C(O)-, -C(O)O-, -C(O)NR-, -O-, -S-, or -NR-;
Z is a direct bond, -O-, or -C(O)NR-;
R² is -(CH₂)ₘ, -RNC(O)(CH₂)ₙC(O)NR-, -(CH₂)ₚC(O)NH(CH₂)_{q}- or -C(O)(CH₂)ₛC(O)-;
R³ is -(CH₂)ₜ-;
each occurrence of R is independently selected from the group consisting of hydrogen, OH, alkyl, alkenyl, alkynyl, alkoxy, acyl, carboxyl, carboxyl ester, cycloalkyl, cycloalkenyl, heterocycloalkyl, heterocycloalkenyl, aryl, and heteroaryl;
R' is an oligosaccharide, preferably an oligosaccharide with 2 to 6 monosaccharide units, with terminal α-galactosyl moiety at the non-reducing end;
wherein m, n, p, q, s, and t are each independently an integer of 1-6;
and wherein
a is from 50 to 1200; and r is from 0.12 to less than 0.23;
with the proviso that said compound is not any of:
- a compound wherein X' is -S-, R¹ is alkoxy, X is -S-, R² is - (CH₂)ₘ-, wherein "m" is 3, Y is -C(O)NR-, wherein R is hydrogen, R³ is -(CH₂)ₜ-, wherein "t" is 3, Z is a direct bond, R' is linear B-2 trisaccharide (Galα1-3Galβ1-4GlcNAc, CAS No. 101627-01-4), a is 250 and r is 0.16; or
- a compound wherein X' is -S-, R¹ is alkoxy, X is -S-, R² is - (CH₂)ₘ-, wherein "m" is 3, Y is -C(O)NR-, wherein R is hydrogen, R³ is -(CH₂)ₜ-, wherein "t" is 6, Z is a direct bond, R' is galactose-α-1,3-galactose, a is 1050 and r is 0.21.

2. The polymeric glycoconjugate compound according to claim 1, wherein r is from 0.12 to 0.22, preferably from 0.15 to less than 0.21, more preferably from 0.18 to less than 0.21.

3. The polymeric glycoconjugate compound according to any of claims 1 or 2, for use as a medicament.

4. A composition comprising the polymeric glycoconjugate compound according to any of claims 1 or 2, preferably wherein said composition is a pharmaceutical composition further comprising a pharmaceutically acceptable excipient, carrier or vehicle.

5. The polymeric glycoconjugate compound according to any of claims 1 or 2, or the composition according to claim 4, for use in the treatment of a disease mediated by anti-αGal antibodies.

6. A polymeric glycoconjugate compound comprising a terminal alpha-galactosyl moiety; wherein said compound comprises a poly-L-lysine backbone and is represented by formula (I): 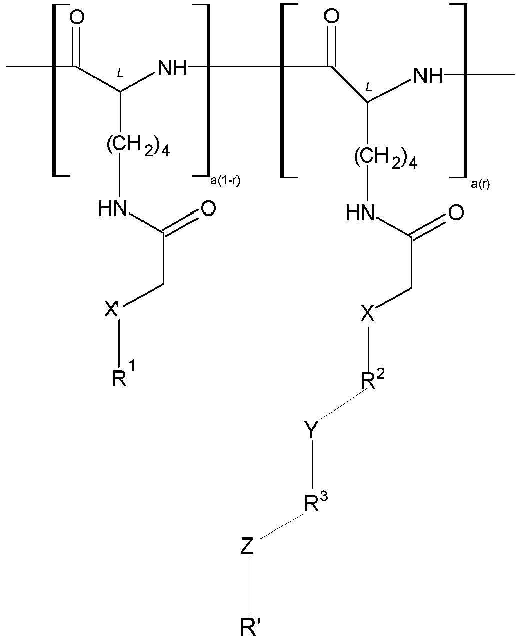
wherein R¹ is hydrogen, alkyl, alkenyl, alkynyl, alkoxy, acyl, amine, carboxyl, carboxyl ester, cycloalkyl, cycloalkenyl, heterocycloalkyl, heterocycloalkenyl, aryl, and heteroaryl;
X, X' and Y are each independently -C(O)-, -C(O)O-, -C(O)NR-, -O-, -S-, or -NR-;
Z is a direct bond, -O-, or -C(O)NR-;
R² is -(CH₂)ₘ, -RNC(O)(CH₂)ₙC(O)NR-, -(CH₂)ₚC(O)NH(CH₂)₄- or -C(O)(CH₂)ₛC(O)-;
R³ is -(CH₂)ₜ-;
each occurrence of R is independently selected from the group consisting of hydrogen, OH, alkyl, alkenyl, alkynyl, alkoxy, acyl, carboxyl, carboxyl ester, cycloalkyl, cycloalkenyl, heterocycloalkyl, heterocycloalkenyl, aryl, and heteroaryl;
R' is an oligosaccharide, preferably an oligosaccharide with 2 to 6 monosaccharide units, with terminal α-galactosyl moiety at the non-reducing end;
wherein m, n, p, q, s and t are each independently an integer of 1-6;
wherein
a is from 50 to 1200; and r is from 0.12 to 1; and
wherein said compound is for use in a method of treating a disease, disorder or condition where anti-αGal IgE antibodies have been described to be involved in its onset, development, or progression (an anti-αGal IgE antibodies-mediated disease) in a subject.

7. The polymeric glycoconjugate compound for use according to claim 6, wherein r is from 0.12 to 0.3; preferably from 0.12 to 0.29, more preferably from 0.15 to 0.28, even more preferably from 0.18 to 0.27.

8. The polymeric glycoconjugate compound according to any of claims 1 or 2, or the polymeric glycoconjugate compound for use according to any of claims 6 or 78, wherein "a" is from 160 to 1200, preferably from 400 to 1200, more preferably from 600 to 1200, even more preferably from 900 to 1100.

9. The polymeric glycoconjugate compound according to any of claims 1 or 2, or the polymeric glycoconjugate compound for use according to any of claims 6 to 8, wherein X is -S-, R² is - (CH₂)₃-, Y is -C(O)NH- or -C(O)O-.

10. The polymeric glycoconjugate compound according to any of claims 1 or 2, or the polymeric glycoconjugate compound for use according to any of claims 6 to 9, wherein said terminal alpha-galactosyl moiety is selected from the group comprising or consisting of galactose-α-1,3-galactose (Galα1-3Gal), linear B-2 trisaccharide (Galα1-3Galβ1-4GlcNAc, CAS No. 101627-01-4), linear B-6 trisaccharide (Galα1-3Galβ1-4Glc, CAS No. 56038-36-9), α1-3 galactobiosyl β-methyl glycoside; α1-3, β1-4 galactotriose (Galal-3Galβ1-4Gal, CAS No. 56038-36-9), galactotetraose (Galα1-3Galβ1-4Galα1-3-D-Gal, CAS No. 56038-38-1), Galili pentasaccharide (L537, Gal-α1,3Gal-β1,4GlcNAc-β1,3Gal-β1,4Glc, CAS No. 119502-59-9), Galα1-3Galβ1-3(Fucα1-4)GlcNAc (#GLY076), Galal-3Galβ1-4(Fucα1-3)GlcNAc (#GLY075), Galα1-3[Galβ1-4GlcNAcβ1-3]4Galβ1-4Glc (#GLY079), Galα1-3[Galβ1-4GlcNAcβ1-3]3Galβ1-4Glc (#GLY078), Galα1-3Galβ1-4Glc (#GLY070), Galα1-3[Galβ1-4GlcNAcβ1-3]2Galβ1-4Glc (#GLY077), Galα1-3Galβ1-4GlcNAcβ1-3Galβ1-4Glc (#GLY071), Galα1-3Galβ1-4GlcNAc, and Galα1-3Galβ1-3GlcNAc (#GLY74-1), preferably wherein said terminal alpha-galactosyl moiety comprises or consists of terminal Galα1-3Galβ1-4GlcNAc.

11. The polymeric glycoconjugate compound according to any of claims 1 or 2, or the polymeric glycoconjugate compound for use according to any of claims 6 to 10, wherein X' is -S-, R¹ is -CH₂CH(OH)CH₂OH, X is -S-, R² is - (CH₂)₃-, Y is -C(O)NH-, R³ is - (CH₂)₃-, Z is a direct bond, and R' is linear B-2 trisaccharide (Galα1-3Galβ1-4GlcNAc, CAS No. 101627-01-4).

12. The polymeric glycoconjugate compound for use in a method according to any of claims 6 to 11, wherein said disease mediated by anti-aGal IgE antibodies is an allergic response in a subject sensitized to αGal.

13. The polymeric glycoconjugate compound for use according to claim 12, wherein said allergic response mediated by anti-aGal immunoglobulins of the IgE isotype occurs further to exposure to a protein with a glycosylation pattern containing the αGal epitope by the oral or parenteral route.

14. The polymeric glycoconjugate compound for use according to any of claims 6 to 13, wherein further to administration of the compound to the subject said compound removes at least 80%, preferably at least 90% of the serum anti-aGal immunoglobulins of the IgE isotype.

15. The polymeric glycoconjugate compound for use according to any of claims 6 to 14, wherein said compound is formulated for parenteral administration, preferably for intravenous, intramuscular or subcutaneous administration, more preferably for subcutaneous administration.
